# EUROPEAN PATENT APPLICATION

(11) **EP 4 487 864 A1**
(43) Date of publication of application: **08.01.2025**
(21) Application number: 23183690.9
(22) Date of filing: 05.07.2023
(51) Int. Cl.: A61K 38/17, A61K 31/365, A61K 31/4706, A61P 1/00, A61P 17/02, G01N 33/50

(54) **COMPOSITIONS FOR THE PREVENTION OR TREATMENT OF A DISEASE THAT IS ASSOCIATED WITH A WEAKENED INTESTINAL BARRIER**

(71) Applicant: Universitätsmedizin der Johannes Gutenberg-Universität Mainz, 55131 Mainz (DE)
(72) Inventor: REINHARDT, Christoph, 55129 Mainz (DE); KOLLAR, Bettina, 65187 Wiesbaden (DE); PONTAROLLO, Giulia, 55128 Mainz (DE); KIOUPTSI, Klytaimnistra, 64295 Darmstadt (DE); MANN, Amrit, 55120 Mainz (DE)
(74) Representative: Patentanwälte Dr. Keller, Schwertfeger Partnerschaft mbB

(57) **Abstract**

The present invention relates to a composition, comprising neuropilin-1 (NRP-1) and/or a compound that maintains or upregulates intestinal epithelial NRP-1 protein levels and promotes Hedgehog (Hh) signaling in the intestinal epithelium for use in the prevention or treatment of a disease that is associated with a weakened intestinal barrier. Furthermore, the present invention relates to an in-vitro method for the detection of a weakened intestinal barrier in a mammal, comprising the steps of providing a sample of the intestinal epithelium from a donor mammal, measuring the protein or mRNA levels of epithelial neuropilin-1 (NRP-1) in epithelium cells isolated from said sample and correlating the observed NRP-1 levels with the levels observed in control samples from healthy donors, wherein lower NRP-1 levels in the sample from the donor mammal compared to the control samples indicate the presence or predisposition of a weakened intestinal barrier in said mammal.

## Description

### FIELD OF INVENTION

The present invention relates to compositions, comprising neuropilin-1 (NRP-1) and/or a compound that maintains or upregulates intestinal epithelial NRP-1 protein levels and promotes Hedgehog (Hh) signaling in the intestinal epithelium. The compositions of the present can be used for the prevention or treatment of a disease that is associated with a weakened intestinal barrier. Administration of neuropilin-1 (NRP-1) and/or a compound that maintains or upregulates intestinal epithelial NRP-1 protein levels and promotes Hedgehog (Hh) signaling in the intestinal epithelium results in strengthening of the intestinal barrier, and hence results in a prevention or treatment of disorders that are associated with a disturbed intestinal barrier.

### BACKGROUND OF THE INVENTION

The intestinal barrier is the first line of defense against pathogens and food antigens (Paone, P., Cani, P.D. Mucus Barrier, Mucins and Gut Microbiota: The Expected Slimy Partners? Gut 2020, 69, 2232-2243). The tight junction structures, transporters and the like are responsible for the intestinal barrier function to prevent the entry of foreign substances. In a healthy organism, the intestinal barrier is characterized by selective permeability, which means that water, ions and low-molecular substances can freely pass through the barrier, while it is impermeable to macromolecules, toxins, food allergens and pathogens (Thoo, L., Noti, M. Krebs, P. Keep Calm: The Intestinal Barrier at the Interface of Peace and War. Cell Death Dis. 2019, 10, 1-13). When these substances leave the intestine, the immune system is over-activated, resulting in the induction of inflammation, which, if it persists for a long time, leads to serious health consequences (Paone, P., Cani, P.D. Mucus Barrier, Mucins and Gut Microbiota: The Expected Slimy Partners? Gut 2020, 69, 2232-2243).

There is evidence that breakdown of the protective mucosal barrier of the gut plays a central role in colorectal cancer (CRC) development (Genua F, Raghunathan V, Jenab M, Gallagher WM, Hughes DJ. The Role of Gut Barrier Dysfunction and Microbiome Dysbiosis in Colorectal Cancer Development. Front Oncol. 2021 Apr 15;11:626349). Inflammation and oxidative stress in the colonic epithelium are involved in colorectal carcinogenesis and the breakdown of the integrity of the colonic barrier, which may increase the exposure of colonocytes to toxins from the colonic milieu, enhancing inflammatory processes and release of Reactive Oxygen Species (ROS). Obesity and western diet may also alter gut microbiota, degrade the integrity of the gut protective barrier, and expose colonocytes to toxins. A strong and intact gut barrier is therefore essential for the prevention in the treatment of variety of inflammatory bowel diseases and colon cancer. Therefore, maintaining the integrity of the intestinal barrier is one of the most important aspects of ensuring health.

The intestinal barrier consists of the mucus layer, intestinal microbiota, intestinal epithelial cells (IECs), and lamina propria (Takiishi, T., Fenero, C.I.M., Câmara, N.O.S. Intestinal Barrier and Gut Microbiota: Shaping Our Immune Responses throughout Life. Tissue Barriers 2017, 5, e1373208). To maintain the integrity of the intestinal barrier, intestinal epithelial cells and microbiota are essential. The epithelial cells provide a physical barrier to prevent substances from leaking out of the intestinal lumen (Vancamelbeke, M., Vermeire, S. The Intestinal Barrier: A Fundamental Role in Health and Disease. Expert Rev. Gastroenterol. Hepatol. 2017, 11, 821-834). The microbiota is the most unstable element of the intestinal barrier since its composition depends on many factors, such as diet, lifestyle and medications (Yang, MDiet and Gut Microbiota Interaction-Derived Metabolites and Intrahepatic Immune Response in NAFLD Development and Treatment. Biomedicines 2021, 9, 1893). The role of the microbiota in preserving the intestinal barrier largely depends on the fact that commensal microbes adhere to the intestinal mucosa and constitute an additional protective layer. In addition, intestinal bacteria, by taking up space on the surface of the IECs, prevent the adhesion of pathogens, thus protecting the IECs from their invasion and damage (Pickard, J.M.; Zeng, M.Y.; Caruso, R.; Núñez, G. Gut Microbiota: Role in Pathogen Colonization, Immune Responses, and Inflammatory Disease. Immunol. Rev. 2017, 279, 70-89). Furthermore, intestinal bacteria produce antimicrobial substances that inhibit the multiplication of pathogens and secrete mucous substances that create a dense environment in which the translocation of substances is difficult (lacob, S.; lacob, D.G.; Luminos, L.M. Intestinal Microbiota as a Host Defense Mechanism to Infectious Threats. Front. Microbiol. 2019, 10, 3328). Thus, maintaining the integrity of the intestinal barrier requires cooperation between all its components.

The intestinal barrier as well as intestinal epithelial renewal are regulated by pattern recognition receptor signaling pathways and specifically by Toll-like receptors (TLRs). Intestinal growth and differentiation are tightly controlled by feedback signaling loops that transmit signals between the epithelium and the mesenchyme (Yeung, T.M., Chia, L.A., Kosinski, C.M. & Kuo, C.J. Regulation of self-renewal and differentiation by the intestinal stem cell niche. Cell. Mol. Life. Sci. 68, 2513-23 (2011); Büller, N.V., Rosekrans, S.L., Westerlund, J. & van den Brink, G.R. Hedgehog signaling and maintenance of homeostasis in the intestinal epithelium. Physiology 27, 147-55 (2012)).

The Hedgehog (Hh) signaling pathway is a signaling pathway that transmits information to embryonic cells required for proper cell differentiation. The pathway also has roles in the adult. Diseases associated with the malfunction of this pathway include cancer or a variety of inflammatory bowel diseases. The epithelial morphogens Indian and Sonic Hh are engaged in normal gut development. In this intestinal morphogenetic signaling pathway, which signals from the epithelium to the mesenchyme, the Hh precursor proteins Indian Hedgehog and Sonic Hedgehog are expressed by terminally differentiated intestinal epithelial cells and signal to Patched receptors, expressed by subepithelial myofibroblasts and smooth muscle cells (Madison, B.B., Braunstein, K., Kuizon, E., Portman, K., Qiao, X.T., Gumucio, D.L. Epithelial hedgehog signals pattern the intestinal crypt-villus axis. Development 132, 279-89 (2005); Bitgood, M.J. & McMahon, A.P. Hedgehog and Bmp genes are coexpressed at many diverse sites of cell-cell interaction in the mouse embryo. Dev. Biol. 172, 126-38 (1995); van den Brink, G.R., Bleuming, S.A., Hardwick, J.C., Schepman, B.L., Offerhaus, G.J., Keller, J.J. et al. Indian Hedgehog is an antagonist of Wnt signaling in colonic epithelial cell differentiation. Nat. Genet. 36, 277-82 (2004)). Binding to PTCH, the active Hh ligands alleviate the inhibitory effect of the G-protein coupled receptor Smoothened (SMO), in turn activating the zinc-finger glioma-associated oncogene transcription factors (GLI), thus restricting uncontrolled epithelial renewal from the stem cell niche via the regulation of Wnt signaling through bone morphogenetic proteins (BMPs) (Büller, N.V., Rosekrans, S.L., Westerlund, J. & van den Brink, G.R. Hedgehog signaling and maintenance of homeostasis in the intestinal epithelium. Physiology 27, 147-55 (2012); He, X.C., Zhang, J., Tong, W.-G., Tawfik, O., Ross, J., Scoville, D.H., et al. BMP signaling inhibits intestinal stem cell self-renewal through suppression of Wnt-beta-catenin signaling. Nat. Genet. 36, 1117-21 (2004)).

The type I transmembrane glycoprotein Neuropilin-1 (NRP-1) has been unveiled as a positive regulator of Hh signal transduction in cell culture models, acting in a positive feedback circuit (Hillman, R.T., Feng, B.Y., Ni, J., Woo, W.-M., Milenkovic, L., Hayden Gephart, M.G., et al. Neuropilins are positive regulators of hedgehog signal transduction. Genes Dev. 25, 2333-2346 (2011); Pinskey, J.M., Franks, N.E., McMellen, A.N., Giger, R.J. & Allen, B.L. Neuropilin-1 promotes hedgehog signaling through a novel cytoplasmic motif. J. Biol. Chem. 292, 15192-15204 (2017)). It has been shown that NRP-1 expressed by colon adenocarcinoma cells promotes tumor angiogenesis (Parikh, A.A., Fan, F., Liu, W.B., Ahmad, S.A., Stoeltzing, O., Reinmuth, N., et al. Neuropilin-1 in colon cancer: expression, regulation, and role in induction of angiogenesis. Am. J. Pathol. 164, 2139-2151 (2004)). In endothelial cells, NRP-1 is a well-established co-receptor of angiogenic vascular endothelial growth factor receptor-2 (VEGFR2) signaling and it is a receptor for class 3 semaphorins (SEMA3), influencing both angiogenesis and neuronal axon guidance (Soker, S., Takashima, S., Miao, H.Q., Nufeld, G., Klagsburn, M. Neuropilin-1 is expressed by endothelial and tumor cells as an isoform-specific receptor for vascular endothelial growth factor. Cell 92, 735-745 (1998); Kolodkin, A.L., Levengood, D.V., Rowe, E.G., Tai, Y.T., Giger, R.J., Ginty, D.D. Neuropilin is a semaphorin III receptor. Cell 90, 753-762 (1997)). Although the Hh pathway is pivotal for gut development, it is presently unknown whether this pathway is modulated by epithelial NRP-1 and the commensal gut microbiota in the small intestine *in vivo* and how this affects intestinal physiology at steady-state conditions.

Compositions for improving the intestinal barrier are known. For example, WO2019131767A1 describes a composition for improving intestinal barrier function by the use of flavan-3-ol polymers. US2020315998A1 describes a composition for improving intestinal barrier function, comprising gallic acid as an active ingredient. JP2020007255A describes an intestinal barrier function-improving agent, characterized by containing shrimp seeds.

### DESCRIPTION OF THE INVENTION

Against this background, it is the object of the present invention to provide alternative agents that are suitable to strengthen the intestinal barrier in order to prevent or treat diseases that are associated with a weakened intestinal barrier.

This object is solved by a composition comprising the features of claim 1. Preferred embodiments are claimed in the sub-claims.

The present invention is based on the surprising finding that neuropilin-1 (NRP-1) is a positive feedback regulator of intestinal Hh signaling. The inventors found that commensal microbiota weakens the intestinal barrier by suppressing epithelial NRP-1 and Hh signaling. As shown herein, elevated epithelial NRP-1 levels resulted in a strengthened gut barrier, revealing a role of epithelial NRP-1 signaling in the regulation of the intestinal barrier function through postnatal control of Hh signaling. The invention therefore suggests to administer or deliver external NRP-1 to a subject in need thereof in order to increase epithelial NRP-1 levels in the intestine. Raising low levels of NRP-1 in the intestinal epithelium to normal levels found in healthy controls improves gut barrier function. Alternative embodiments relate to compounds that have the property of increasing internal levels of epithelial NRP-1. Such compounds may increase the expression of NRP-1 protein in the intestinal epithelium. Alternative embodiments suggest to inhibit NRP-1 degradation, NRP-1 downregulation or NRP-1 inactivation, for example by compounds or active agents that inhibit lysosomal degradation of NRP-1. In some embodiments, these compounds are TLR2 inhibitors (TL2-C29) or lysosom inhibitors.

More specifically, the present invention relates to a composition, comprising NRP-1 and/or a compound that maintains or upregulates intestinal epithelial NRP-1 protein levels and promotes Hedgehog (Hh) signaling in the intestinal epithelium for use in the prevention or treatment of a disease that is associated with a weakened intestinal barrier.

As used herein, a "weakened intestinal barrier" or "weakened gut barrier" refers to an increase in intestinal permeability. A weakened intestinal barrier is usually characterized by an increased permeability for certain substances or agents, and has a disturbed function compared to the intestinal barrier found in healthy organisms.

To test whether the intestinal barrier is weakened, common methods in the art can be utilized that assess intestinal permeability. The most common method for in vivo assessment of intestinal permeability is the urinary recovery of administered marker molecules. Other than the detection in urine, most markers can also be retrieved in blood samples. There are also ex-vivo and in-vitro approaches that can be used for assessment of electrical resistance and intestinal flux parameters when biological samples are not available. More detailed analyses of specific changes that may affect the function of the intestinal barrier can be achieved using molecular and histological approaches on intestinal tissue sections. On the other side, measurement of biomarkers in urine, blood or faeces provides a simple and non-invasive method to evaluate intestinal barrier function without the need for previous administration of test molecules. Examples that can be used in the context of the present invention include, but are not limited to urinary biomarkers, biomarkers in serum or plasma, or faecal biomarkers.

The NRP-1 as used in the context of the present invention is any suitable protein, polypeptide or nucleic acid molecule encoding NRP-1, most preferably epithelial NRP-1, as they occur in any mammalian species, preferably in humans. In a preferred embodiment, the NRP-1 component in the composition of the present invention is recombinant NRP-1. The invention comprises any artificial or natural NRP-1 , or homologs of any species. In some embodiments, the NRP-1 is a polypeptide encoding epithelial NRP-1. In some embodiments, the polypeptide encoding NRP-1 is a variant that is longer or shorter than the wildtype equivalent of epithelial NRP-1. The invention also covers insertions, deletions and/or substitutions of one or more amino acids in the amino acid sequence encoding NRP-1.

Artificial NRP-1 may be modified by the addition or deletion of chemical moieties. In a preferred embodiment, the recombinant NRP-1 comprises amino acids 890-902 within the cytoplasmic domain and the cytoplasmic guanosine triphosphatase-activating protein domain of plexins. The presence of these structural elements is required for NRP-1 function.

A compound of the invention that maintains or upregulates epithelial NRP-1 protein levels and that promotes cell-intrinsic Hh signaling has the property of inhibiting or preventing NRP-1 degradation, NRP-1 inactivation or NRP-1 downregulation. In some embodiments, the compound that inhibits NRP-1 degradation is selected from the group consisting of TLR2 inhibitors, lysosomal inhibitors, chloroquine or bafilomycin A1.

In some embodiments, the compound is an effective agent that inhibits or prevents Hh signaling suppression. In some embodiments, the composition comprises a compound that activates Hh signaling. In some embodiments, compound is an inhibitor that inhibits or prevents NRP-1 degradation, NRP-1 inactivation or NRP-1 downregulation. In preferred embodiments, the inhibitor is selected from the group consisting of TLR agonists, peptidoglykan, macrophage-activating lipopeptide-2, Pam2CSK4, Smoothened antagonist GDC-0449 (vismodegib); cyclopamine, Hh antagonist CUR61414 (Smoothened); Forskolin (Gli1). The compounds can also be used, either alone or in any combination, to achieve a fine adjustment or modulation of the Hh pathway.

In a preferred embodiment, the compound that inhibits or prevents NRP-1 degradation, NRP-1 inactivation or NRP-1 downregulation is selected from the group consisting of antisense-oligonucleotide, siRNA, shRNA, non-coding RNA (ncRNA), miRNA, long non-coding RNA (IncRNA) or aptamer. In preferred embodiments, aptamers are used delivery agents of compounds that have the property of inhibiting NRP-1 degradation or NRP-1 downregulation. For example, aptamers of siRNA can be used for targeting mRNA expression of a compound that inhibits NRP-1 degradation, NRP-1 downregulation or NRP-1 inactivation. Small interfering RNA (siRNA) as used in the context of the present invention suppress the expression of genes that downregulate NRP-1 by targeting mRNA expression. Targeted delivery of siRNA to specific cells is highly desirable for safe and efficient RNAi-based therapeutics. As a result of the action of these inhibitory compounds, epithelial NRP-1 expression will be maintained, enhanced or no longer adversely affected (e.g., by NRP-1 degradation or NRP-1 downregulation).

Examples of protein targets that are used to prevent NRP-1 degradation, NRP-1 inactivation or NRP-1 downregulation include, but are not limited to Glioma-associated oncogene homolog 1 (GLI1), Indian Hedgehog (IHH), Sonic Hedgehog (SHH), Patched-1 (PTCH1), Patched-2 (PTCH2), Hedgehog Interacting protein (HHIP), Bone Morphogenetic Protein-4 (BMP4), Bone Morphogenetic Protein-7 (BMP7), Bone Morphogenetic Protein-2 (BMP2), BOC Cell Adhesion Associated (BOC), Smoothened (SMO), Fibroblast Growth Factor 9 (FGF9), Niemann-Pick C1 Protein (NPC1), Hedgehog Acyltransferase (HHAT).

Examples of nucleic acid targets that are used to prevent NRP-1 degradation, NRP-1 inactivation or NRP-1 downregulation include, but are not limited to Glioma-Associated Oncogene Homolog 1 (Gli1) , Indian Hedgehog (Ihh), Sonic Hedgehog (Shh), Patched-1 (Ptch1), Patched-2 (Ptch2), Hedgehog Interacting protein (Hhip), Bone Morphogenetic Protein-4 (Bmp2), Bone Morphogenetic Protein-7 (Bmp7), Bone Morphogenetic Protein-2 (Bmp2), BOC Cell Adhesion Associated (Boc), Smoothened (Smo), Fibroblast Growth Factor 9 (Fgf9), Niemann-Pick C1 Protein (Npc1), Hedgehog Acyltransferase (Hhat).

In alternative embodiments, the compound that inhibits or prevents NRP-1 degradation, NRP-1 downregulation or NRP-1 inactivation is an antibody or antibody fragment. Such an antibody or antibody fragment recognizes an epitope on the surface of a compound that is involved in NRP-1 degradation, NRP-1 downregulation or NRP-1 inactivation. As a result, NRP-1 degradation, NRP-1 downregulation or NRP-1 inactivation will be prevented.

In some preferred embodiments, the compositions of the present invention are formulated as probiotics, prebiotics or synbiotics.

As used herein "probiotics" refer to living microorganisms that have benefits for humans when ingested in sufficient quantities.

As used herein, "prebiotics" refers to any substance, bacteria or mixture thereof that helps to maintain a healthy balance in the intestinal microbiome. Usually, prebiotics fosters growth or activity of beneficial microorganisms in the gut, there they alter the composition of organisms in the gut microbiome.

As used herein, "synbiotics" refer to a mixture comprising of live microorganisms and substrates selectively utilized by host microorganisms that confers a health benefit on the host.

In preferred embodiments, the probiotics, prebiotics or synbiotics contain bacteria or microorganisms that activate Hh signaling. In other embodiments, the probiotics, prebiotics or synbiotics contain bacteria or microorganisms that ameliorate microbiota-induced suppression of Hh signaling and/or reduction of intestinal epithelial NRP-1 protein levels.

In some embodiments, the prebiotics, probiotics or synbiotics may contain lactic acid bacteria, bifidobacteria, dietary fiber, polysaccharides and the like. The lactic acid bacteria and bifidobacteria are preferably bacteria which can be taken orally. The dietary fiber may be any of water-insoluble dietary fiber and water-soluble dietary fiber. Examples of water-insoluble dietary fibers include cellulose, lignin, hemicellulose, wheat bran, apple fiber, sweet potato fiber, chitin and the like. Water-soluble dietary fibers are roughly classified into high-viscosity substances and low-viscosity substances, and examples of high-viscosity substances include pectin, konjac mannan, alginic acid, sodium alginate, guar gum, agar and the like.

In preferred embodiments, the prebiotics, probiotics or synbiotics comprises a mixture of eight bacteria that form the altered Schaedler flora (ASF). Preferably the mixture comprises ASF 356 Clostridium sp., ASF 360 Lactobacillus intestinalis, ASF 361 Lactobacillus murinus, ASF 457 Mucispirillium schaedleri, ASF 492 Eubacterium plexicaudatum, ASF 500 Pseudoflavonifractor sp., ASF 502 Clostridium sp., and ASF 519 Parabacteroides goldsteinii. As shown by the present invention, ASF colonization affects mRNA expression of Hh pathway genes. A mixture of these bacteria therefore promotes Hh signaling, and hence contributes to the strengthening of the intestinal barrier by maintaining epithelial NRP-1 function.

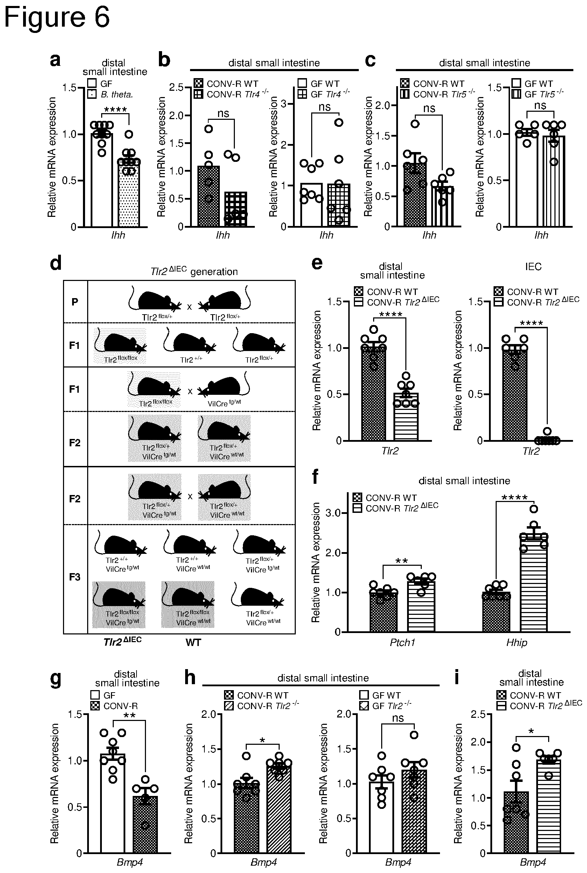

In some embodiments, the composition of the invention can comprise Bacteroides thetaiotaomicron. Bacteroides thetaiotaomicron has been shown to suppress the Hh pathway (Figure 6a). By addition of this bacterium to the composition, a fine adjustment in concert with other components in the composition is possible. For example, an increased Hh signaling response of a compound can attenuated by using compounds that counteract these effects.

In some other embodiments, the composition can comprise Bacteroides fragilis, Bacillus subtilis, Akkermansis muciniphila, Roseburia intestinalis and/or Lactobacillus acidophilus.

The invention also covers embodiments that relate to a combination of Hh signalingenhancing compounds and bacteria or microorganisms that modulate the Hh pathway. By using a combination of individual components, it is possible to modulate Hh signaling and hence regulate NRP-1 expression.

In some embodiments, the composition of the present invention is provided in form of a nutritional supplement, food ingredient or dietary supplement.

In preferred embodiments, the compositions of the present invention are formulated as pharmaceutical compositions, comprising NRP-1 or a compound that maintains or upregulates epithelial NRP-1 protein levels and promotes Hh signaling, and at least one pharmaceutically acceptable carrier, diluent, adjuvant and/or excipient.

Pharmaceutical compositions suitable for use in the present invention include compositions wherein NRP-1 or the active compounds are contained in an effective amount to achieve the intended purpose. For example, an effective amount of NRP-1 or a compound that improves NRP-1 expression or prevents NPR-1 degradation may be that amount that strengthens the intestinal barrier. Determination of effective amounts and measuring the permeability of the intestinal barrier is well within the capability of those skilled in the art.

The pharmaceutical compositions of the present invention may be administered in a number of ways depending upon whether local or systemic treatment is desired and upon the disease to be treated. Administration may be oral, enteral, topical (including rectal delivery) or parenteral. Oral administration provides for no proteolytic degradation of the active ingredient in the stomach and prevents an inadequate absorption mechanism for polypeptides from the intestinal lumen. Parenteral administration includes intravenous, intraarterial, subcutaneous, intraperitoneal or intramuscular injection or infusion. Alternatively, intracranial, e.g., intrathecal or intraventricular, administration can be applied to a subject in need of such a treatment.

Compositions and formulations for parenteral, intrathecal or intraventricular administration may include sterile aqueous solutions that may also contain buffers, diluents and other suitable additives such as, but not limited to, penetration enhancers, carrier compounds and other pharmaceutically acceptable carriers or excipients.

Pharmaceutical compositions of the present invention include, but are not limited to, solutions, emulsions, and liposome-containing formulations. These compositions may be generated from a variety of components that include, but are not limited to, preformed liquids, self-emulsifying solids and self-emulsifying semisolids.

Pharmaceutical formulations for parenteral administration include aqueous solutions of the active compounds in water-soluble form. Suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Pharmaceutical preparations can be obtained by combining the active compounds with solid excipient, optionally grinding a resulting mixture, and processing the mixture of granules, after adding suitable auxiliaries, if desired, to obtain tablets or dragee cores. Suitable excipients are carbohydrate or protein fillers such as sugars, including lactose, sucrose, mannitol, or sorbitol; starch from corn, wheat, rice, potato, etc.; cellulose such as methyl cellulose, hydroxypropylmethyl-cellulose, or sodium carboxymethylcellulose; and gums including arabic and tragacanth; and proteins such as gelatin and collagen. If desired, disintegrating or solubilizing agents may be added, such as the cross-linked polyvinyl pyrrolidone, agar, alginic acid or a salt thereof such as sodium alginate.

The pharmaceutical formulations of the present invention are preferably presented in unit dosage form and may be prepared according to conventional techniques well known in the pharmaceutical industry.

The compositions of the present invention may be formulated into any of many possible dosage forms such as, but not limited to, tablets, capsules, liquid syrups, soft gels, suppositories, and enemas. The compositions of the present invention may also be formulated as suspensions in aqueous, non-aqueous or mixed media. Aqueous suspensions may further contain substances that increase the viscosity of the suspension including, for example, sodium carboxymethylcellulose, sorbitol and/or dextran. The suspension may also contain stabilizers.

In some embodiments, the pharmaceutical compositions of the present invention are delivered by any suitable carrier or vehicle, including but not limited to nanoparticles, liposomes, dendrimers or micelles. In some embodiments, the active compound of the composition is delivered by means of a viral vector system such as adenoviruses or helper-dependent adenoviral vectors (HdAd). The active compound is provided in any suitable form that is required for the delivery vehicle. For example, NRP-1 mRNA or cDNA can be provided in full-length or truncated form and my contain one or more regulatory sequences that control NRP-1 protein expression.

For parenteral administration, the NRP-1 will be preferably employed in an amount within the range of from about 0.001 mg/kg to about 10 mg/kg or more (e.g., 20, 30, 40, 50 or more mg/kg) and preferably from about 0.01 mg/kg to about 1 mg/kg. The compositions of the present invention may be administered in the dosage forms as described above in single or divided doses of one to multiple times daily, or continuously or semi-continuously.

For recombinant NRP-1 or any active compound used in the compositions of the invention, the therapeutically effective dose can be estimated initially from cell culture assays. Then, preferably, dosage can be formulated in animal models (particularly murine models) to achieve a desirable circulating concentration range.

A therapeutically effective dose refers to that amount which ameliorates or prevents signs and/or symptoms of a disease state or condition. In more particular, a therapeutically effective dose refers to that amount that results in strengthening of the intestinal barrier or normalizing intestinal barrier function in the subject to be treated.

As used herein, the term "subject" refers to a patient that is administered the therapeutic composition comprising the compounds of the present invention. Examples of subjects, include, but are not limited to, humans and other mammals such as non-human primates, farm animals, horses, dogs, and cats.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index, and it can be expressed as the ratio LD50 /ED50. Compounds which exhibit large therapeutic indices are preferred. The data obtained from these cell culture assays and additional animal studies can be used in formulating a range of dosage for human use. The dosage of such compounds lies preferably within a range of circulating concentrations that include the ED50 with little or no toxicity. The dosage varies within this range depending upon the dosage form employed, sensitivity of the patient, and the route of administration.

In the context of NRP-1 it may be necessary to determine the most effective amount that results in a strengthening of the intestinal barrier without initiating adverse side effects such as promoting tumor growth or tumor angiogenesis. The exact dosage may be chosen by a subject's physician in view of the patient to be treated. Dosage and administration are adjusted to provide sufficient levels of the active compound (i.e., NRP-1 or any other active compound that modulates permeability of the intestinal barrier) to maintain the desired effect, i.e., to maintain the function of the intestinal barrier. Additional factors that may be taken into account include the severity of the disease state; age, weight, and gender of the patient; diet, time and frequency of administration, drug combination(s), reaction sensitivities, and tolerance/response to therapy. Long-acting pharmaceutical compositions might be administered every 3 to 4 days, every week, or once every two weeks depending on half-life and clearance rate of the particular formulation.

In some preferred embodiments, a composition comprising NRP-1 or a compound that maintains or upregulates intestinal epithelial NRP-1 protein levels and promotes Hedgehog (Hh) signaling in the intestinal epithelium is administered to a subject via a transanal route (e.g., via an enema).

In some embodiments, the pharmaceutical composition may be provided as a salt and can be formed with many acids, including but not limited to hydrochloric, sulfuric, acetic, lactic, tartaric, malic, succinic.

In preferred embodiments, the disease that is associated with a weakened intestinal barrier is an inflammatory bowel. As used herein, "inflammatory bowel disease" refers to any of a variety of diseases characterized by inflammation of all or part of the intestines, preferably selected from the group consisting of ulcerative colitis, Crohn's disease, celiac disease, diverticulitis, pouchitis, proctitis, mucositis after chemotherapy and radiotherapy, radiation induced enteritis, chronic nonspecific colitis.

In alternative embodiments, the disease that is associated with a weakened intestinal barrier is a tumor disease selected from the group consisting of colorectal carcinoma. A colorectal carcinoma refers to any type of colon cancer, bowel cancer or rectal cancer.

The pharmaceutical compositions of the present invention have the capability to ameliorate the effect of commensal microbiota which have been shown to weaken the intestinal barrier by suppressing epithelial neuropilin-1 and Hh signaling. By supplementing NRP-1 or by promoting NRP-1 expression in the epithelial intestine, epithelial NRP-1 protein levels can be maintained at functional levels. More specifically, the intestinal barrier function can be improved by using compositions of the present invention comprising NRP-1. In alternative embodiments, the composition comprises a compound that modulates NRP-1 expression or prevents NRP-1 degradation, downregulation or inactivation. In preferred embodiments, the active compound that is used for improving the intestinal barrier function is an analog or a homolog of epithelial NRP-1, or derivatives thereof. The present invention thus contributes to the prevention or amelioration of a condition or disease associated with an abnormal intestinal barrier function.

The present invention also relates an in-vitro method for the detection of a weakened intestinal barrier in a mammal, comprising the steps:
a. providing a sample of the intestinal epithelium from a donor mammal,
b. measuring the protein or mRNA levels of epithelial neuropilin-1 (NRP-1) in epithelium cells isolated from said sample,
c. correlating the observed NRP-1 levels with the levels observed in control samples from healthy donors, wherein lower NRP-1 levels in the sample from the donor mammal compared to the control samples indicate the presence or predisposition of a weakened intestinal barrier in said mammal.

In a preferred embodiment, the sample of the intestinal epithelium is a sample from the small intestinal epithelium.

The invention also relates kits for the detection of a weakened intestinal barrier by providing means for measuring the protein or mRNA levels of epithelial NRP-1 in intestinal epithelium cells in a sample obtained from a mammal donor.

### BRIEF DESCRIPTION OF THE FIGURES

Figure 1. Hedgehog pathway is modulated by the gut microbiota via epithelial TLR2.
Figure 2. NRP-1 protein levels in the gut epithelium are regulated by the gut microbiota through TLR2-mediated lysosomal degradation.
Figure 3. Impairment of gut barrier function by the gut microbiota, gut epithelial neuropilin-1-deficiency, and inhibition of hedgehog signaling.
Figure 4. Deficiency of epithelial NRP-1 reduces density of blood capillaries without affecting lacteal length in the distal small intestine.
Figure 5. Impact of commensals on hedgehog signaling, neuropilin-1 and intestinal epithelial permeability.
Figure 6. Indian Hedgehog expression in monocolonized and TLR-knockout mouse models; generation and characterization of conditional intestinal epithelial *Tlr2* knockout mouse model; Bone morphogenetic protein 4 regulation by gut microbiota through TLR2.
Figure 7. NRP-1 and NRP-2 expression in the gut of GF, CONV-D, and CONV-R mice, and MODE-K cell culture model.
Figure 8. Characterization of the *Nrp1*^{ΔIEC} mouse model, efficacy of NRP-1-deficiency, NPR2 expression and microbiome analysis.
Figure 9. VEGF-A expression in the small intestine of the *Nrp1*^{ΔIEC} mouse line and characterization of blood capillaries and lacteals in the distal small intestine of GDC-0449-treated mice.
Figure 10. ASF colonization affects mRNA expression of Hh pathway genes.

### EXAMPLES

The invention will be further illustrated in the following examples. By no means shall the invention be restricted to these specific examples. The invention also covers variations, modifications and combinations of different features or embodiments, even if not explicitly described.

As shown in the following examples, the present invention reveals a role for the commensal microbiota and epithelial NRP-1 signaling in the regulation of intestinal barrier function through postnatal control of Hh signaling. The invention reveals that elevated epithelial NRP-1 levels can be associated with a strengthened gut barrier. Functionally, intestinal epithelial cell-specific NRP-1-deficiency (*NRP-1^{ΔIEC}*) results in decreased Hh pathway activity and a weakened gut barrier.

The invention provides proof for microbiota-triggered TLR signaling in the small intestinal epithelium in the postnatal control of the Hh pathway and the regulation of the intestinal barrier. Epithelial NRP-1 has been identified as a critical element that in the absence of gut commensals augments Hh signaling, thus supporting the integrity of the gut epithelial barrier. By colonization with gut microbiota, epithelial cell surface expression of NRP-1 was abolished via TLR2 signaling, promoting NRP-1 degradation. Unexpectedly, epithelial deficiency of NRP-1 is linked to decreased vascularization of small intestinal villus structures. In line with strengthened gut barrier function and increased epithelial NRP-1 levels of germ-free (GF) mice, the tissue-specific epithelial deficiency of NRP-1 expression resulted in the microbiota-dependent suppression of Hh signaling, linked to a weakened gut epithelial barrier.

### Commensals suppress morphogenetic hedgehog signals via TLR2

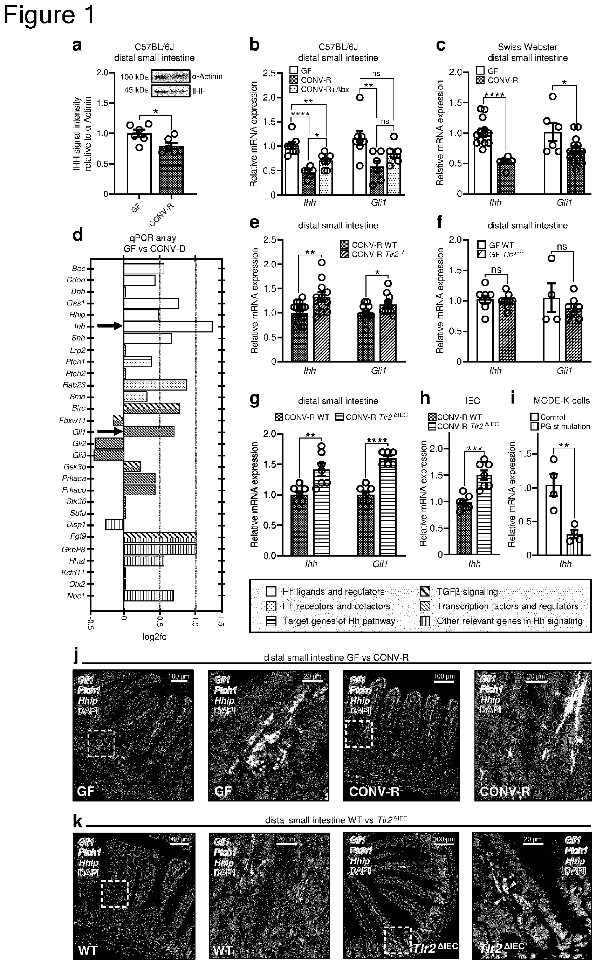

To understand microbiota-triggered signaling mechanisms affecting intestinal homeostasis, it was analyzed whether the gut microbiota has an impact on the Hh pathway. IHH protein is expressed by terminally differentiated enterocytes, whereas *Ihh* mRNA expression is highest in the crypt villus junctions³³. Interestingly, Western blot analyses on small intestinal tissue lysates of adult mice revealed that the major hedgehog morphogen IHH is elevated on the protein level at germ-free housing conditions as compared to conventionally raised (CONV-R) counterparts (**Fig, 1a**). Both in C57BL/6J and Swiss Webster mice, transcript levels of *Ihh* were markedly reduced in the small intestine of CONV-R mice relative to GF housing conditions (**Fig. 1b****, c**). The influence of gut commensals on Hh signaling was further corroborated by reduced expression of the Hh target gene *Gli1* in CONV-R mice as compared to GF controls (**Fig. 1b****, c**). As a confirmation, antibiotic-induced decimation of commensals significantly increased Hh transcripts and reversed the microbiota-dependent decrease of Hh signaling, reflecting the reversible and dynamic microbiota-dependent regulation of this morphogenetic signaling pathway (**Fig. 1b**). In line with these findings, monocolonization of GF mice with the common gut symbiont *Bacteroides thetaiotaomicron* was sufficient to dampen small intestinal *Ihh* expression **(****Fig. 6a****).** As the presence of gut microbiota consistently attenuated the Hh signaling axis, a qPCR array analysis was performed comparing GF mice with conventionalized mice (conventionally-derived, CONV-D; GF mice colonized with the cecal microbiota from a CONV-R mouse for two weeks). This analysis confirmed suppression of various elements of the Hh pathway by colonization with gut microbiota, including *Ihh* and *Gli1* **(****Fig. 1d****).** Collectively, these results demonstrate a dynamic microbiota-triggered suppression of the intestinal Hh pathway.

Next it was determined by which regulatory pathway the colonization with gut commensals might trigger the suppression of Hh signaling. Since GF mouse models and antibiotics-induced microbiota-depletion have revealed that the expression levels of epithelial TLRs in the ileum, in particular TLR2, TLR4, and TLR5, are tightly controlled by the gut microbiota, it was investigated whether microbiota-induced TLR signaling could be involved in dampening intestinal Hh gradients. To this end, global *Tlr2*-deficient (*Tlr2*^{*-*/*-*})*, Tlr4*-deficient (*Tlr4*^{*-*/*-*})*,* and TIrS-deficient (*Tlr5*^{*-*/}*⁻)* mice were re-derived as GF and the small intestinal Hh signals were compared with their CONV-R counterparts. It could be shown by the present invention that CONV-R *Tlr2*^{*-*/*-*} mice exhibited increased expression of *Ihh* and *Gli1* compared to CONV-R wild-type (WT) controls, whereas the expression levels did not differ in GF *Tlr2*^{*-*/*-*} or GF WT mice (**Fig. 1e****, f**). In contrast to *Tlr2*^{*-*/*-*} mice, the *Ihh* expression levels in the distal small intestine of *Tlr4*^{*-*/*-*} and *Tlr5*^{*-*/*-*} mice were not significantly changed by the presence of gut microbiota (**Fig**. **6b****, c**). Thus, the results of the present invention indicate that small intestinal Hh signals are primarily suppressed through microbiota-triggered TLR2 signaling.

To pinpoint if TLR2 signaling in the gut epithelial compartment is sufficient to elicit suppression of Hh signaling, as observed in the small intestine of the global *Tlr2* knockout mouse model, a *Tlr2-flox x Villin-Cre* mouse line (*Tlr2^{ΔIEC})* was generated with markedly reduced *Tlr2* expression in the small intestine and a complete absence of *Tlr2* mRNA in intestinal epithelial cells (**Fig**. **6d****, e**)³⁵. As shown, *Ihh* expression levels were significantly increased in the small intestine and in isolated epithelial cells of tissue-specific *Tlr2^{ΔIEC}* mice relative to the Cre-negative *Tlr2*^{*fl*/*fl*} littermate controls, whereas transcript levels of the Hh target *Gli1* were elevated in the small intestine (**Fig**. **1g****, h**). In addition, elevated expression levels of the Hh target genes *Patched-1* (*Ptch1*) and *Hh interacting protein (Hhip)* further support the suppressive role of epithelial TLR2 signaling **(****Fig. 6f****).** Conversely, stimulation of the mouse small intestinal epithelial cell line MODE-K³⁶ with the TLR2/6 agonist peptidoglycan (PG) led to a marked reduction of *Ihh* transcript levels **(****Fig. 1i****).** Coherent with epithelial-to-mesenchymal signaling of the identified microbiota-TLR2-regulated Hh pathway, single molecule fluorescence *in situ* hybridization (sm-FISH), comparing GF with CONV-R, and *Tlr2^{ΔIEC}* mice with WT-floxed littermates, showed that Hh targets *Gli1, Ptch1,* and *Hhip* were exclusively expressed in the intravillus mesenchyme (**Fig**. **1j****, k**)^{22,23}. Underscoring epithelial-to-mesenchymal signaling of the identified signaling axis^{37,38}, the mRNA expression of the hedgehog target gene *Bmp4*²³*,* which is specifically expressed in the intravillus mesenchyme^{39,40}, was likewise suppressed via microbiota-triggered epithelial TLR2 signaling **(****Fig. 6g****-i).** Collectively, the results of the present invention reveal TLR2 signaling in the intestinal epithelium as a major signaling hub, connecting the colonization with gut commensals with the adaptation of epithelial Hh signaling gradients.

### Gut epithelial NRP-1 is suppressed by microbiota-driven TLR2

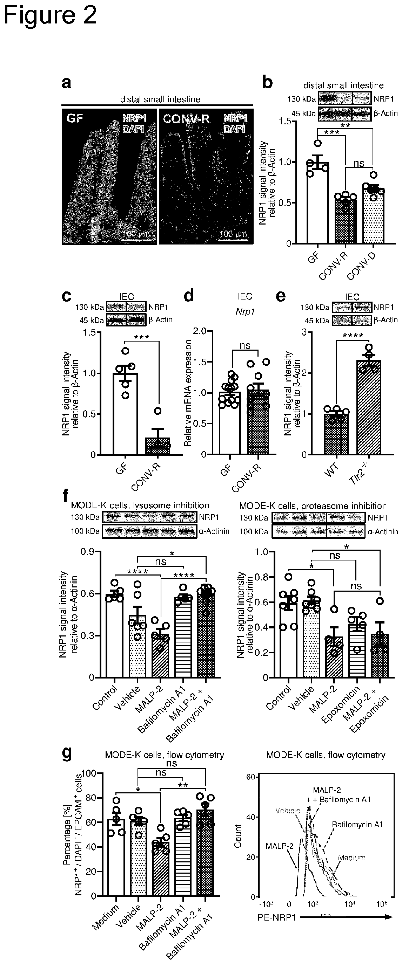

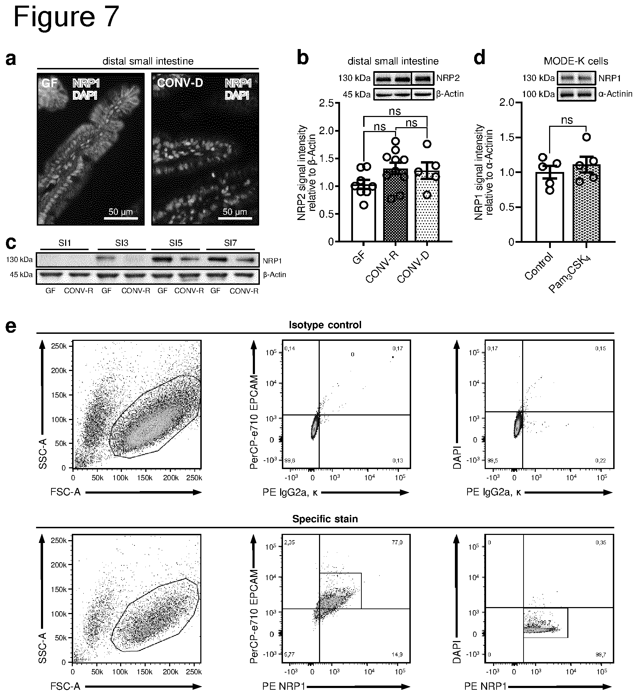

Next it was analyzed whether the gut microbiota impacts expression levels of the Hh-pathway regulator NRP-1 in the epithelial lining of the distal small intestine *in vivo.* Immunofluorescence staining of fixed-frozen small intestine sections showed high NRP-1 protein expression in terminally differentiated epithelial cells of GF mice compared with CONV-R controls, indicating the microbiota-dependent regulation of this receptor protein (Fig. 2a). Epithelial NRP-1 protein levels were likewise reduced in colonized mice (CONV-D) (Fig. 7a). Immunoblot analyses confirmed reduced NRP-1 protein levels in the distal small intestine of colonized mice, showing a significant reduction of NRP-1 in CONV-R and CONV-D mice as compared to GF controls (**Fig. 2b**). In contrast to NRP-1, protein levels of the homologue NRP-2 in the distal small intestine were not microbiota-regulated (**Fig. 7b**). A reduction in NRP-1 protein levels in CONV-R as compared to GF housing conditions was consistently found along the entire length of the small intestine, from jejunum to ileum **(****Fig. 7c****).** This pronounced reduction in NRP-1 protein levels in colonized mice was further corroborated with isolated small intestinal epithelial cells, demonstrating that the presence of microbiota suppressed epithelial NRP-1 protein expression without affecting transcript levels **(****Fig. 2c****, d).** In conclusion, the results of the present invention show that colonization with gut microbiota yields in reduced protein levels of the Hh-regulator NRP-1 in primary small intestinal epithelium by posttranscriptional mechanisms.

In accordance with the microbiota-induced suppression of the Hh pathway **(****Fig. 1a****-f),** the reduced epithelial protein levels of the Hh pathway-regulator NRP-1 are a result of the recognition of microbial patterns by TLR2, as NRP-1 protein levels were strongly increased in primary small intestinal epithelial cells of *Tlr2*^{*-*/*-*} mice **(****Fig. 2e****).** In line, the diacyl lipopeptide TLR2/6 agonist macrophage-activating lipopeptide-2 (MALP-2) efficiently suppressed NRP-1 protein levels in the epithelial MODE-K cell culture model, as demonstrated by Western blot and flow cytometry analyses **(****Fig. 2f****, g).** In contrast, stimulation of MODE-K cells with the TLR2/1-specific agonist Pam₃CSK₄, a synthetic triacylated lipopeptide, did not change NRP-1 protein levels **(****Fig. 7d****).** Next, the mechanism by which NRP-1 is degraded was further investigated. Inhibitor treatments revealed that NRP-1 is downregulated by a TLR2/6-induced lysosomal pathway. MODE-K cells that were stimulated with MALP-2 following pre-incubation with the lysosomal inhibitor bafilomycin A1, did not show reduced NRP-1 protein levels **(****Fig. 2f****,** left panel). In contrast, the MALP-2-induced reduction in NRP-1 protein levels was not prevented by blockade of proteasomal degradation with the inhibitor epoxomicin **(****Fig. 2f****,** right panel). Of note, the inhibitor experiments with bafilomycin A1 on MODE-K cells were independently confirmed by flow cytometry analysis, demonstrating the blockade of MALP-2-triggered reduction of NRP-1 cell surface levels via the lysosomal pathway **(****Fig. 2g** and **Fig. 7e****).** Collectively, the analyses of the present invention revealed that NRP-1 in the small intestinal epithelium is downregulated by the commensal microbiota and TLR2 signaling through the lysosomal degradation pathway.

### NRP-1 is critical for microbiota-dependent gut barrier control

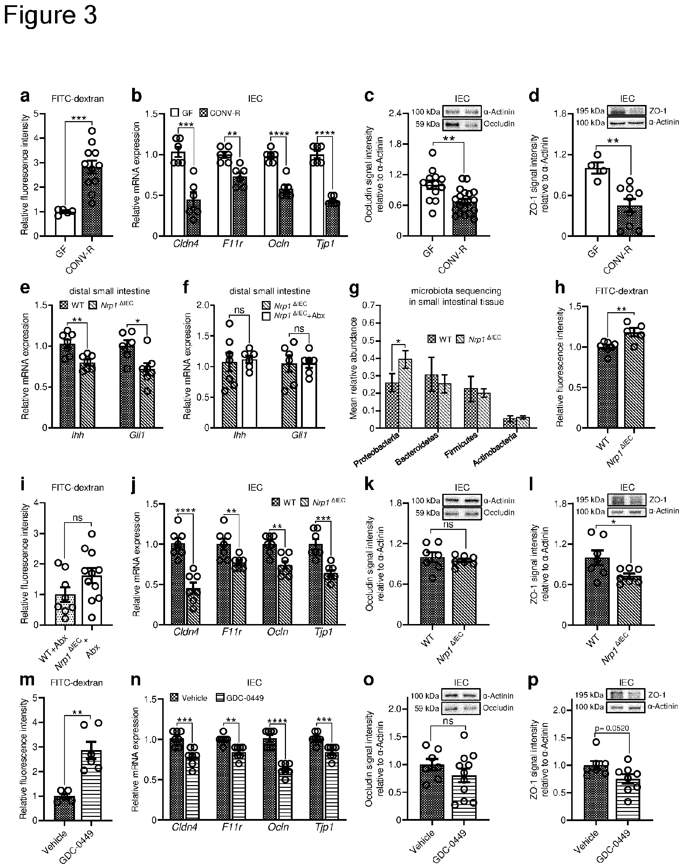

The commensal gut microbiota is a key factor in the regulation of the intestinal epithelial barrier, which substantially depends on epithelial tight junction complexes. By intragastric administration of FITC-dextran to GF and CONV-R mice, it was demonstrated by the present invention that CONV-R mice have reduced paracellular intestinal epithelial barrier function compared with their GF counterparts, with 3-times lower fluorescence intensity detected in the plasma of GF mice **(****Fig. 3a****).** Enhanced gut barrier at GF housing conditions was further supported by increased mRNA expression of the tight junction proteins claudin-4 *(Cldn4),* junctional adhesion molecule-A (*F11r*), occludin *(Ocln),* and zonula occludens-1 (ZO-1, tight junction protein-1, *Tjp1)* in isolated small intestinal epithelial cells **(****Fig. 3b****).** In line, the protein levels of occludin and ZO-1 were diminished in the intestinal epithelial cells of CONV-R mice relative to GF counterparts **(****Fig. 3c****, d).** Thus, the results of the present invention highlight that the gut microbiota weakens the gut epithelial barrier.

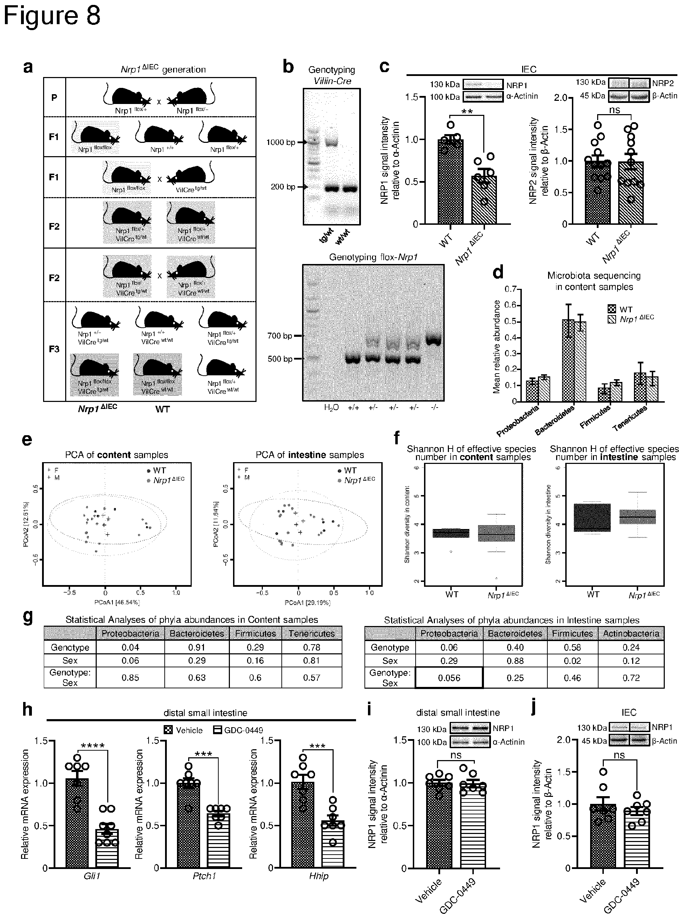

To functionally explore whether the increased intestinal permeability in CONV-R mice could be attributed to the microbiota-triggered reduction in NRP-1 in the gut epithelial lining **(****Fig. 2a****-c),** a mouse line with enterocyte-specific *NRP-1* deficiency (*NRP-1^{ΔIEC}*) was generated **(****Fig. 8a****, b).** As expected, deficiency of intestinal epithelial NRP-1 did not affect NRP-2 protein levels **(****Fig. 8c****).** This model allowed to investigate whether the microbiota-induced impairment of epithelial NRP-1 protein levels may be involved in the weakened intestinal epithelial barrier. In accordance with the microbiota-dependent regulation of the Hh pathway **(****Fig. 1a****-e),** *NRP-1^{ΔIEC}* mice displayed significantly reduced small intestinal mRNA levels of *Ihh* and the Hh-target *Gli1* but showed unaltered *Ihh* and *Gli1* transcripts when the gut microbiota was depleted by treatment with an antibiotic cocktail containing ampicillin and neomycin **(****Fig. 3e****, f).** In line with reduced epithelial NRP-1 protein levels **(****Fig. 2a****-c),** and increased gut permeability due to reduced expression of tight junctional components in CONV-R mice **(****Fig. 3a****-d),** *NRP-1^{ΔIEC}* mice indeed showed impaired intestinal barrier function, as determined by increased FITC-dextran levels in the serum **(****Fig. 3h****).** Antibiotic treatment abolished these differences in gut epithelial barrier function **(****Fig. 3i****).** The barrier defect observed in *NRP-1^{ΔIEC}* mice was further substantiated by consistently reduced epithelial *Cldn4, F11r, Ocln,* and *Tjp1* transcripts and significantly reduced ZO-1 protein levels, whereas occludin was unaffected **(****Fig. 3j****-l).** Interestingly, the impaired gut barrier function of *NRP-1^{ΔIEC}* mice was associated with an increased abundance of Proteobacteria in small intestinal tissue **(****Fig. 3g****),** an association also observed in Crohn's disease. This difference was not significant in the small intestinal content **(****Fig. 8d****).** Moreover, principal component analysis (PCA) and Shannon index did not show major differences between the two groups **(****Fig. 8e****-g).** Thus, the results of the present invention identify epithelial NRP-1 as a regulator of microbiota-influenced gut barrier function, associated with an altered gut microbial diversity.

### Hedgehog suppression weakens the gut epithelial barrier

Since the gut microbiota suppresses Hh pathway activity via lysosomal degradation of the Hh regulator NRP-1 in the intestinal epithelium **(****Fig. 1 a-c** and **Fig. 2 a-c),** it was next addressed whether the Hh pathway is involved in the regulation of gut barrier function. Since mice with a targeted deletion of *Ihh* are embryonically lethal, a pharmacological inhibition of the pathway was used by *in* vivo-treatment of C57BL/6J mice with the Smoothened (Smo)-antagonist GDC-0449 (Vismodegib). In line with the microbiota-dependent reduction in Hh signaling (**Fig**. **3f****, g**) and impaired gut barrier function observed in *NRP-1^{ΔIEC}* mice (**Fig**. **3h**, **j**, **l**), the inhibition of tonic Hh signaling with GDC-0449 strongly weakened the epithelial gut barrier (**Fig. 3m**). The efficacy of the pharmacological *in vivo* inhibition by GDC-0449 was confirmed by reduced small intestinal transcript levels of the Hh targets *Gli1, Ptch1,* and *Hhip* (**Fig. 8h**). Of note, GDC-0449 inhibition did not feed-back to reduce NRP-1 protein levels, neither in distal small intestinal tissue nor in isolated epithelial cells (**Fig**. **8i****, j**). The GDC-0449 inhibitor treatment demonstrated that impaired gut barrier function caused by suppression of the Hh pathway is due to reduced gut epithelial mRNA expression of several epithelial junction constituents **(****Fig. 3n****).** In accordance with the gut epithelial deficiency of the positive Hh-regulator NRP-1, when the Hh signaling pathway was blocked, epithelial protein levels of the tight junction components occludin and ZO-1 were reduced (**Fig**. **3o****, p**). In summary, the results of the present define Hh pathway regulation through microbiota-suppressed epithelial NRP-1 as a critical epithelial permeability-regulating factor.

### Epithelial NRP-1 deficiency impairs capillary network formation

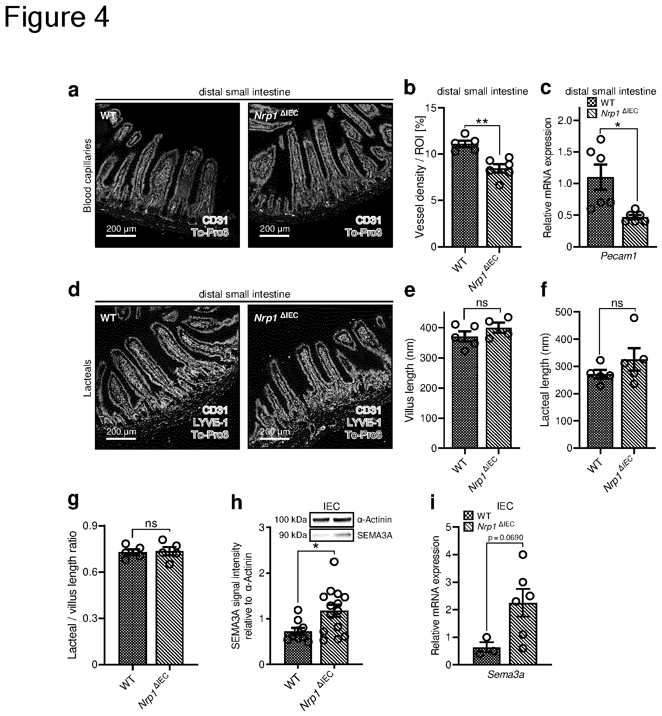

Fluorescence staining for the pan-endothelial cell marker PECAM-1 (CD31) revealed a significant reduction in the density of blood capillaries in small intestinal villus structures of *NRP-1^{ΔIEC}* mice relative to Cre-negative WT littermate controls (**Fig**. **4a****, b**). This finding was further confirmed by the observation of reduced *Pecam1* mRNA expression in the small intestine of *NRP-1^{ΔIEC}* mice **(****Fig. 4c****).** In contrast, epithelial NRP-1-deficiency did not compromise villus length, lacteal length, or the lacteal per villus length ratio, as assessed by staining for the lymphatic marker LYVE-1 **(****Fig. 4d****-g).**

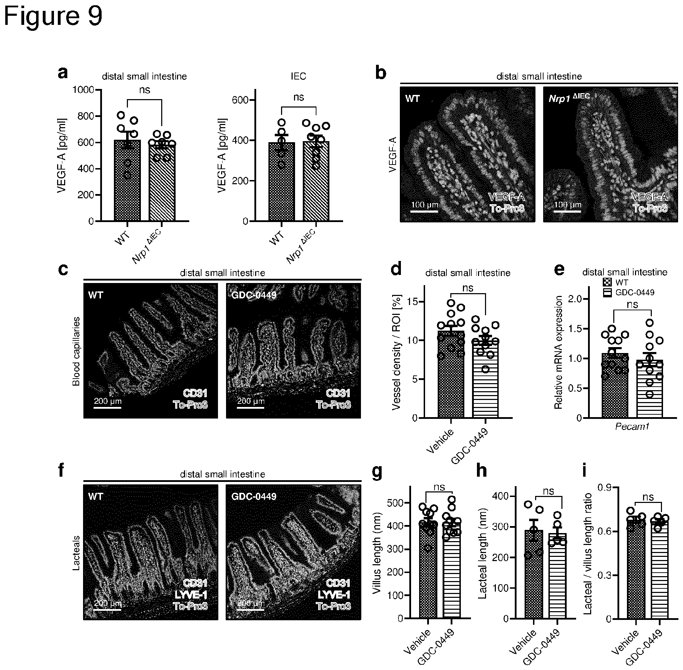

To explain this vascular phenotype, the gut epithelial levels of the NRP-1 ligand VEGF-A, assisting VEGFR2-mediated angiogenesis, and SEMA3A, an established NRP-1 ligand that limits angiogenesis, was analyzed. Unexpectedly, VEGF-A was unaffected by intestinal epithelial NRP-1-deficiency, as shown by ELISA analysis of small intestinal tissue lysates and isolated epithelium, as well as by immunofluorescence staining **(****Fig. 9a****, b).** In contrast, SEMA3A, an inhibitor of epithelial cell migration and an established vascular morphogen, was elevated in intestinal epithelial cells of *NRP-1^{ΔIEC}* mice, both at the mRNA and protein level (**Fig. 4h****, i**). Hence, reduced villus vascularization in *NRP-1^{ΔIEC}* mice likely results from anti-angiogenic SEMA3A-NRP-1 signaling. In contrast to the identified role of Hh signaling in epithelial gut permeability regulation **(****Fig. 3m****-p**), *in vivo* inhibition of the Hh pathway with GDC-0449 did neither affect villus vascularization nor lacteal length **(****Fig. 9c-****i).** In summary, the results of the present invention uncovered NRP-1, expressed by small intestinal epithelial cells, as an important regulator of gut mucosal vascularization.

### Summary of the Results

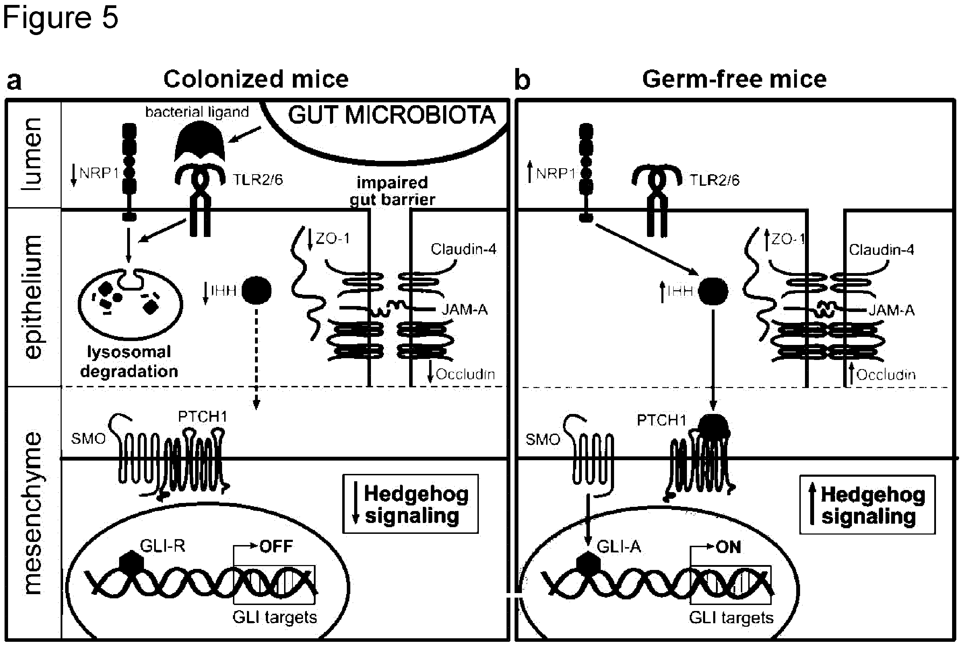

Taken together, the present invention reveals that the gut microbiota suppresses tonic Hh signaling in the small intestine, thus regulating intestinal barrier function **(****Figure 5**). As shown by the present invention, the Hh pathway activity is primarily suppressed through microbiota-triggered TLR2/6 signals in the gut epithelium and identified intestinal epithelial NRP-1 as a pivotal microbiota-dependent Hh regulator, which contributes to stabilize the gut epithelial barrier. The present invention uncovers a microbiota-driven morphogenetic signaling pathway that links intestinal innate immune receptor signaling to Hh pathway activity. This microbiota-host interaction tunes the epithelial gut barrier and thus most likely has broad consequences on small intestinal nutrient uptake and intestinal immune homeostasis.

The results of the present invention define the small intestinal epithelial TLR2 as a target of the gut microbiota that downregulates epithelial-derived Ihh gradients and downstream signaling in the lamina propria. Dependent on TLR2 activation of lysosomal degradation, NRP-1 on the gut epithelium is efficiently suppressed by commensals to cease the positive feedback circuit with the Hh pathway. Based on NIH-2T3 mouse fibroblast cell culture models stimulated with exogenous Hh ligands, cell-intrinsic role for NRP-1, acting as a positive feedback regulator of the Hh pathway in mesenchymal cells, has been demonstrated. This cell-intrinsic signaling function of NRP-1 critically depends on a 12 amino acid region within the cytoplasmic domain of the receptor (amino acids 890-902) and the cytoplasmic guanosine triphospatase-activating protein domain of multiple plexins promoting cell-intrinsic Hh signaling. The *in vivo* analyses of the present invention indicate that NRP-1 expressed by the gut epithelial lining regulates microbiota-dependent expression of *Ihh,* the major small intestinal agonist situated upstream in the intestinal epithelial-to-mesenchymal Hh signaling axis, which is exclusively expressed by the gut epithelium but not the mesenchyme. Epithelial Hh, acts on its targets, such as PTCH1, SMO, in the mesenchyme. Since *Bmp4* is absent in the gut epithelium but expressed at high levels in the intravillus mesenchyme, the results of the present invention confirm the epithelial-to-mesenchymal axis of the identified signaling cue by microbiota-TLR2-instructed suppression of the Hh target gene *Bmp4.*

Notably, the uncovered microbiota-induced suppression of the epithelial NRP-1/Hh signaling axis has broad implications for the microbiota's impact on gut barrier function. Increased paracellular permeability, mRNA downregulation of tight junction constituents, and a consistent reduction of epithelial ZO-1 were observed in colonized mice, in the absence of epithelial NRP-1, and after the inhibition of the Hh pathway. Thus, based on experimentation with germ-free mice, tissue-specific knockout mouse models and inhibitor treatments, the present invention established a mechanistic link between host colonization status and epithelial gut barrier integrity. Furthermore, the analyses underlying the present invention revealed that epithelial deficiency of NRP-1 impairs villus blood capillary formation in the distal small intestine. In conclusion, the present invention unravels the functional relevance of epithelial NRP-1 as a microbiota-dependent regulatory factor of epithelial Hh signaling in the regulation of the gut epithelial barrier and mucosal vascular remodeling.

### Material and Methods

### Animals

Germ-free (GF) C57BL/6J Swiss Webster mice and the conventionally-raised (CONV-R) controls originated from the colonies of Fredrik Bäckhed (Wallenberg Laboratory, Gothenburg, Sweden). GF and CONV-R distal small intestinal tissues from *Tlr5*^{*-*/*-*} mice were obtained through collaboration with Benoit Chassaing and Andrew Gewirtz (Georgia State University, Atlanta, USA). *Tlr2*^{*-*/*-*} mice (B6;129-Tlr2<tm1Kir>/J-M; stock #004650) were purchased from Jackson Laboratories (Bar Harbor, MA, USA) and *Tlr4*^{*-*/*-*} mice were provided by Prof. Markus Radsak (Department of Medicine III, University Medical Center Mainz, Germany). *Tlr2*^{*-*/*-*} and *Tlr4*^{*-*/*-*} mouse strains were rederived as GF by aseptic hysterectomy and maintained in sterile conditions as a GF mouse colony in flexible film isolator systems. The GF status of mice was tested weekly by PCR for detection of bacterial 16S rDNA and by bacterial culture of feces. *NRP-1-flox* mouse line (B6.129(SJL)-NRP-1 <tm2Ddg>/J; stock #005247) and the Villin-Cre mouse line were purchased from Jackson Laboratories. The epithelial knockout mouse models Tlr2-flox x VilCre (*Tlr2*^{ΔIEC}) and NRP-1-flox x VilCre (*NRP-1*^{ΔIEC}*)* were generated by crossing with the B6.Cg-Tg(Vil1-cre)1000Gum/J line (stock #021504). All animals were 8 to 14-weeks old male or female mice housed in the Translational Animal Research Center (TARC) of the University Medical Center Mainz under specific-pathogen-free (SPF, CONV-R) conditions in EU type II cages with two to five cage companions with standard autoclaved lab diet and water *ad libitum,* 22°C ± 2°C room temperature, and a 12-h light/dark cycle, 55-65% humidity. All groups of mice were age- and sex-matched and free of clinical symptoms.

For conventionalization experiments (CONV-D mice), the cecum content of two age-matched CONV-R animals (one male and one female) was re-suspended in 10 ml PBS, and a 200 µl-aliquot was given to GF mice by oral gavage. The conventionalization was allowed to proceed for two weeks. For antibiotics treatment (CONV-R + Abx), an antibiotic cocktail (1.5 g/l ampicillin; 0.5 g/l neomycin) was added to the drinking water for two weeks. Mice were euthanized by CO₂ inhalation, followed by cervical dislocation.

### GDC-0449 treatment

50 mg GDC-0449 (Cat #S1082, Selleck Chemicals, Houston, USA) were dissolved in 8.3 ml of 0.5% (w/v) methylcellulose (Cat #8421.1 , Carl Roth, Karlsruhe, Germany), 0.2% (v/v) tween-80 (Cat 9139.1, Carl Roth) and 0.5% (v/v) DMSO (Cat #276855, Sigma-Aldrich, St. Louis, USA), thus obtaining a 6 mg/ml stock solution. Each day, for 7 days, the mice were given an aliquot of the GDC-0449 suspension or the vehicle by oral gavage (30 mg/kg body weight). 4 hours before the gavage, the drinking water was removed from the cage. On the last day, 4 hours after the gavage, mice were sacrificed and organs were extracted.

### Organ harvest

After euthanasia, the abdominal cavity was excised and the small intestine was extracted, cleaned from adipose tissue and flushed from mucous and fecal content with ice-cold PBS (Cat #BP399, Fisher BioReagents, Waltham, USA). For whole tissue analysis, a segment of the distal small intestine was snap frozen in liquid nitrogen or, in the case of histological analysis, was fixated in ROTIHistofix 4.5% (Cat #2213.3, Carl Roth) for 24 hours before paraffin embedding.

Primary intestinal epithelial cells (IEC) were isolated as detailed (Stzepourginski, I, et al. CD34+ mesenchymal cells are a major component of the intestinal stem cells niche at homeostasis and after injury. Proc. Natl. Acad. Sci. USA. 114, E506-13 (2017)). Briefly, the PBS-cleaned distal small intestine was cut-open lengthwise, and incubated with 5 ml of 10 mM EDTA (Cat #A4892,0500, AppliChem GmbH, Darmstadt, Germany) in PBS under agitation (250 rpm, 30 min, 37°C). After manual shaking (removal of the epithelial layer), IEC were harvested (6,500 rpm, 5 min, 4°C), washed in PBS and re-suspended in the proper buffer according to total RNA or protein extraction.

### MODE-K cell culture

MODE-K cells were purchased from Inserm-U1111 (Dr. Kaiserlian, Lyon, France). In brief, cells were maintained at 37°C in a humidified atmosphere of 5% CO₂ with RPMI 1640 Medium, GlutaMAX (Cat #11554516, Gibco, Thermo Fisher Scientific, Waltham, USA) supplemented with 100 mM sodium pyruvate (Cat #11360070), 1 M HEPES (Cat #15630080), 1% (v/v) non-essential amino acids (Cat # 11140050), 50 mM 2-Mercaptoethanol (Cat #21985023), 10% (v/v) heat-inactivated fetal bovine serum (FBS, Cat #10082147) (all from Thermo Fisher Scientific), and 0.2% (v/v) penicillin-streptomycin (Cat #P4333, Sigma Aldrich, St Louis, USA). For stimulation, MODE-K cells were seeded in 6-well plates until they reached 80-90 % confluence, following by treatment in cell culture medium for 2 h with 0.125% (v/v) bafilomycin A1 (Cat #SML1661, Sigma Aldrich), 0.1% (v/v) epoxomicin (Cat #324801, Sigma Aldrich), 0.5 µM Pam₃CSK₄ (Cat #506350, Sigma Aldrich), or 2 µg/ml MALP-2 (Cat #ALX-162-027-C050, Enzo Life Sciences, Farmingdale, USA). For the vehicle, the equivalent amount of DMSO (0.1-0.125% (v/v)) (Cat #A3672,0050, AppliChem) was used. MODE-K cells were manually detached with a cell scraper for Western Blot, followed by protein extraction. For flow cytometry analysis, cells were re-suspended.

### Flow cytometry of MODE-K cells

MODE-K cells were harvested and re-suspended in ice-cold 1X PBS added with 3% (v/v) FBS (Cat #10082147 Thermo Fisher Scientific), from now on, FACS Buffer. Next, MODE-K cells were pre-incubated with 1:100 (v/v) rat anti-mouse CD16/32 TruStain FcX mAb (clone 93, Cat #101319, BioLegend, San Diego, USA) for 10 min on ice. After pelleting (300 xg, 10 min, 4°C) and washing in FACS buffer, MODE-K cells were incubated with fluorescent antibodies (Ab) for 30 min at 4°C, protected from light. The following Ab and dilutions were used: 1:20 (v/v) EPCAM (CD326)-PerCP-eFluor 710 (clone G8.8, Cat #46-5791, Thermo Fisher Scientific); 1:100 (v/v) NRP-1 (CD304)-PE (clone 3E12, Cat #145203), and 1:100 (v/v) IgG2a, κ Isotype Control (clone RTK2758, Cat #400501), both from Biolegend. After washing with ice-cold PBS (300 × g, 10 min, 4°C), the cells were re-suspended in 500 µl PBS and analysed by flow cytometry on a BD FACSCanto II instrument (BD Biosciences, Franklin Lakes, USA). Data were visualized on the BD FACSDiva Software (version 6.1.3) and analysed by FlowJo Software (version 10.5.2) (Ashland, USA). The gating strategy was based on the isotype control.

### Immunofluorescence

Paraffin-embedding of small intestinal sections was performed by the Histology Core Facility (University Medical Center Mainz, Germany). After slicing the embedded tissue in 7 µm-sections using a rotatory microtome (Leica Biosystems, Wetzlar, Germany), sections were deparaffinized in xylol (Cat #251769, AppliChem) and rehydrated in a graded series of ethanol and water as detailed elsewhere. After antigen retrieval by steaming in 10 mM citric acid (Cat #5110, Carl Roth) for 20 min and 2x 5 min washing in PBS-T (PBS + 0.05 % (v/v) tween-20), samples were blocked with protein block solution (Cat #X0909, Agilent, Santa Clara, USA) for 10 min. The following primary antibodies were used overnight at 4 °C: 1:100 (v/v) PECAM-1 rabbit mAb (CD31, clone D8V9E, Cat #77699, Cell Signaling Technology, Danvers, USA), 1:100 (v/v) LYVE-1 rat mAb (clone ALY7, Cat # 14-0443-80, Thermo Fisher Scientific) and 1:100 VEGF-A rabbit mAb (Cat #ab52917, abcam, Cambridge, UK). After 3x 5 min-washes in PBS-T, secondary antibodies were incubated for 1 h protected from light. In particular, 1:500 (v/v) goat anti-rabbit IgG (H+L) conjugated with Alexa Fluor 488 (Cat # 4412, Cell Signaling Technologies) was used to detect PECAM-1 and VEGF-1, whereas 1:500 (v/v) goat anti-rat IgG (H+L) conjugated with Alexa Fluor 555 (Cat #4417, Cell Signaling Technologies) was used against LYVE-1. Samples were washed 3x 5 min in PBS-T, counterstained with To-Pro3 Iodide (Cat #T3605, Thermo Fisher Scientific) for 30 min and subsequently, mounted with faramount (Cat #S3025, Agilent). For fluorescent staining on frozen sections from the distal small intestine of Swiss Webster mice, 6 µm-cryosections were cut and stored at -80°C. On the day of the experiment, samples were thawed at RT for 20 min and samples were blocked for 1 h in TBST (TBS + 0.1 % (v/v) tween-20), added with 10% (v/v) fecal calf serum (FCS, heat-inactivated, Cat #AL2420, Life Technologies GmbH, Darmstadt, Germany). NRP-1 rabbit mAb (clone D62C6, Cat #3725, Cell Signaling Technology) was diluted 1:2000 (v/v) in blocking buffer and added on the slides for 2.5 h at RT. Slides were washed 3 × 10 min in TBST and incubated with 1:1000 (v/v) anti-rabbit secondary antibodies, i.e. goat anti-rabbit IgG (H+L) conjugated with Alexa Fluor 488 (Cat # 4412, Cell Signaling Technologies) or donkey anti-rabbit IgG (H+L) Alexa Fluor 555 (Cat #A-31572, Life Technologies) for 1 h in the dark. Slides were washed 3x 10 min in TBST, and nuclei were counterstained with DAPI (Cat # 32670, Sigma Aldrich). Slides were washed again 3 × 10 min in TBST and mounted with faramount reagent. Images were acquired using a Zeiss LSM 710 microscope, following morphometric analysis employing CellSens Dimension (version 4.1) (Carl Zeiss, Oberkochen, Germany).

### qRT-PCR analyses

Total RNA was isolated from whole tissue with TRlreagent (Cat #T9424, Sigma Aldrich), and from IEC with the Promega Kit (Cat #Z6012, Promega, Madison, USA), according to the manufacturer's instructions. After purity and quality check, mRNA was converted into cDNA with High Capacity cDNA Reverse Transcriptase Kit (Cat #4368814, Applied Biosystems, Waltham, USA). The cDNA was diluted 1:20 (v/v) by RNase-free water (Aqua ad iniectabilia, B. Braun, Melsungen, Germany). Relative mRNA expression was quantified by qPCR analyses on a qTOWER3 Real-Time PCR Thermal Cycler instrument (Analytic Jena AG, Jena, Germany) equipped with the qPCRsoft (version 4.0) software, using iTaq Universal SYBR Green Supermix (Cat #1725121, BioRad Laboratories Inc., Hercules, USA). Each biological sample was analyzed in triplicates, using the ribosomal protein *L32* as the housekeeping gene. For relative expression quantification, cycle threshold (Ct) values were analyzed, according to Pflaffl and normalized for the control group, i.e. GF mice, when different microbiota colonization statuses were compared, or WT mice, in case of the analyses on transgenic mouse strains.

### Single Molecule Fluorescence in Situ Hybridization (sm-FISH)

RNAscope Multiplex Fluorescent V2 assay (Cat #PS-00003027.1, Bio-Techne GmbH, Minneapolis, USA) was performed on 5 µm-paraffin sections (for paraffin embedding and cutting with microtome see above) according to the manufacture's instruction. *Gli1, Ptch1* and *Hhip* transcripts were ordered from the company and probed using TSA Vivid Fluorophore 650, 520, and 570, respectively. Cells nuclei were counterstained with DAPI. Distal small intestine of GF vs. CONV-R and *Tlr2*^{ΔIEC} vs WT floxed littermates were analyzed, using 3 mice per group. The probes were visualized with a confocal microscope (Leica TCS SP8, Wetzlar, Germany). Data analysis was accomplished with the Leica Application Suite X (LAS EZ) software (version 3.7.5.24914).

### qPCR array

RT2 profiler PCR array kit (Cat #PAMM-078Z, Qiagen, Hilden, Germany) was used according to the manufacturer's instructions. RNA was isolated from the distal small intestine of 7 mice per group and adjusted to a concentration of 200 ng/µl. cDNA was synthetized using the RT² First Strand Kit (Cat #330404, Qiagen, Hilden, Germany). Per group, one 96-well plate was used. The obtained data were analyzed using the online software RT2 Profiler PCR Array Data Analysis (version 3.5).

### Western blot

Small intestine tissue, isolated IEC or MODE-K cells were re-suspended in a variable volume of RIPA Buffer (Cat #20-188 Merck Millipore, Burlington, USA) added with protease and phosphatase inhibitor mini-tablets (Cat #A32959, Thermo Fisher Scientific). For small intestinal specimens, mechanical lysis was performed on a TissueLyserII (Qiagen, Hilden, Germany) (2 × 2 min, 30 Hz, with 1 min-pause in between). Protein extraction was allowed to proceed for 30 min on ice. Samples were centrifuged (10.000 x g, 15 min, 4°C) and protein quantification was performed on collected supernatants by DC Protein Assay (Cat #500-0116, BioRad Laboratories Inc.). All samples were diluted to the same concentration and added to 5X home-made reducing Laemmli buffer. Thermic denaturation was promoted at 99°C for 5 min. For Western Blot assays, proteins were separated on an electrophoretic run and transferred on a 0.45 µm PVDF membrane (Cat #IPVH00010, Merck Millipore). The membranes were blocked in 5% (w/v) milk or BSA in TBST (i.e, TBS + 0.1% (v/v) tween-20) and incubated overnight with the primary antibodies (Ab) at 4°C. The following Ab were used: 1:1000 (v/v) IHH (Cat.# ARP45230_T100, Aviva System Biology), 1:1000 (v/v) NRP-1 (Cat #3725S, Cell Signaling Technology), 1:1000 (v/v) NRP-2 (Cat #3366P, Cell Signaling Technology), 1:1000 (v/v) occludin (Cat #OC-3F10, Thermo Fisher Scientific), 1:1000 (v/v) ZO-1 (Cat #61-7300, Thermo Fisher Scientific) and 1:1000 (v/v) SEMA3A (Cat #ab23393, Abcam). 1:2500 (v/v) α-Actinin (Cat #3134S, Cell Signaling Technology) and 1:1000 (v/v) β-Actin (Cat #4970S, Cell Signaling Technology) were used as loading controls. After 3X 10 min-washes in TBST, secondary 1:5000 (v/v) goat anti-rabbit IgG (H+L) (Cat #PI-1000) or 1:2500 (v/v) horse anti-mouse IgG (H+L) (Cat #PI-2000, both from Vector Laboratories, Burlingame, USA), conjugated with HRP, were added for 90 min at RT. Relative protein expression was quantified by ECL (Cat #95538S, Cell Signaling Technology) and visualized on the FusionCapt Advance (Vilber Lourmat). Band densitometry was performed on the instrument software (version 17.01) and normalized for the control group.

### VEGF-A ELISA

Small intestine tissue or IECs were processed as described before to obtain protein lysates. VEGF-A ELISA assay was performed according to manufacturer instructions using the VEGF-A-Cell Lysate Mouse ELISA Kit (Cat # EMVEGFACL, Invitrogen, Thermo Fisher Scientific, Waltham, USA). Raw absorbance values were read on a Dynex Opsys MR Reader (Dynex Technologies, Chantilly, USA), equipped with the Revelation Quicklink software (version 4.25). The standard curve was constructed by plotting the mean absorbance of each standard on the y-axis against the concentration on the x axis and then used for the calculation of the VEGF-A concentration in the samples.

### FITC-dextran gavage

Water bottles were removed from the cages 4 hours before administration of 50 mg FITC-dextran (MW 4000) (Cat #46944, Sigma Aldrich) per 100 g body weight in PBS. After 4 hours, the mice were anaesthetized by i.p. injection of a solution of 5.0 mg/kg body weight midazolam (Ratiopharm, Ulm, Germany), 0.5 mg/kg body weight medetomidin (Pfizer, New York, USA) und 0.05 mg/kg body weight fentanyl (CuraMed Pharma GmbH, Karlsruhe, Germany) in 0.9% NaCl-solution. Therefore, whole blood without anticoagulants was collected by cardiac puncture⁵⁸ and let standing overnight at 4°C to coagulate. On the next morning, serum was obtained by centrifugation (6,500 rpm, 10 min, 4°C) of whole blood, aliquoted and stored at -20°C for further analyses. The serum samples were diluted 1:1 (v/v) with PBS and measured in duplicates by spectrofluorometry (Fluoroskan Ascent FL, Thermo Fisher, or SpectraMax MiniMax 300 Imaging CYtometer, Molecular Devices) using an excitation of 485 nm and an emission wavelength of 528 nm. A standard serially diluted FITC-dextran (0, 125, 250, 500, 1,000, 2,000, 4,000, 6,000, 8,000 ng/ml) was used for quantification. Fluorescence values were normalized to the control group.

### 16S rRNA gene sequencing and processing

Briefly, the hypervariable regions V1-V2 of the 16S rRNA genes were amplified following a dual-indexing approach sequencing on the Miseq Illumina platform. Final sample sizes included 24 intestine tissues (18 *NRP-1*^{ΔIEC} and 6 wild type), and 23 content (18 *NRP-1*^{ΔIEC} and 5 wild type). Initial sequences processing was performed in Mothur (version 1 31.2) where forward and reverse reads were merged using quality score parameter insert =30 and screened for accurate length, base ambiguity and homopolymers. Chimera were detected and removed in Uchime with reference-based method. Subsequently, sequences were classified from the phylum to genus level using the stasta, and final reads were normalized to 4100 reads per sample. Lastly, operational taxonomic units (OTUs) at 97% sequence similarity threshold were clustered in Mothur.

### Statistical analysis

GraphPad Prism 9.3.1 was used for all statistical analyses and graphs except for the 16S rRNA gene sequencing data. Statistical analyses of the metagenomics data were performed in R package version 3.1. Variations in microbiota structure and diversity were assessed across genotypes using several diversity measures, within tissues and content samples, distinctively. First, variation was evaluated in abundance of the most abundant (major) phyla and genera across genotypes using linear mixed effects models with genotype, sex and sex: genotype included as fixed effects and breeding cage as random effect in "nlme" R package. Best models were fitted by maximizing the restricted loglikelihood (REML), and evaluated and validated by i) checking residuals distribution, ii) plotting fitted and residual values, and iii) inspecting residuals and explanatory variables. Additionally, different diversity measures including Shannon and Bray-Curtis were calculated, based on operational taxonomic units (OTUs) at 97% sequence similarity threshold in "vegan" R package. Comparison of Shannon measure across genotypes was assessed through linear mixed effects models as explained above. Bray-Curtis measure was assessed across genotypes using "adonis" function with 10⁵ permutations, and principal coordinate analyses (PCoA).

**Data availability:** 16S rRNA gene sequencing data are accessible in Sequence Read Archive (SRA) under PRJNA936417. Source data are available with this paper.

**Code availability:** The analyses investigating 16S rRNA sequences were carried out following the vignettes for the R package version 2.5-6 or the R package version 3.1-150.

### FIGURES

**Figure 1****. Hedgehog pathway is modulated by the gut microbiota via epithelial TLR2. (a)** Comparative immunoblot analysis of Indian hedgehog (IHH) protein levels of small intestinal tissue lysates from GF vs CONV-R C57BL/6J mice, relative to α-actinin (n=6 vs 6; p=0.0262). The 45 kDa IHH precursor is detected. Insert shows representative Western Blot. **(b,c)** Relative gene expression of *Ihh* and *Gli1* in **(b)** C57BL/6J GF, CONV-R and CONV-R mice treated with antibiotics (CONV-R + Abx) *(Ihh:* n=7 vs 6 vs 7. GF vs CONV-R p<0.0001; GF vs CONV-R + Abx p=0.0045; CONV-R vs CONV-R + Abx p=0.0413. *Gli1:* n= 8 vs 7 vs 6. GF vs. CONV-R p=0.0097) or **(c)** Swiss Webster GF vs CONV-R mice *(Ihh:* n=13 vs 6. p<0.0001; *Gli1:* n=6 vs 14. p=0.0303). **(d)** qRT-PCR array on pooled concentration-adjusted mRNAs of 7 mice per group (n=7 vs 7), showing differential expression of genes involved in hedgehog pathway (see legend box) between GF and conventionalized (CONV-D) mice (relative to CONV-D). *Ihh* and *Gli1* are highlighted with a black arrow. **(e-h)** Relative gene expression of *Ihh* and *Gli1* in WT mice vs *Tlr2*^{*-*/*-*} global knockout mice in **(e)** CONV-R (*lhh:* n=17 vs 11. p=0.0025; *Gli1:* n=12 vs 11. p=0.4181) or **(f)** GF *(Ihh:* n=8 vs 9. *Gli1:* n=4 vs 7) housing conditions, and in **(g,h)** *Tlr2*^{ΔIEC} CONV-R mice in comparison to WT littermates (Distal small intestine. *Ihh:* n=7 vs 7. p=0.0032; *Gli1:* n=7 vs 6. p<0.0001. IEC. *Ihh:* n=7 vs 7. p=0.0003). In panel **b-g,** qRT-PCR analyses were performed on the whole tissue (distal small intestine), whereas for panel **h** on isolated IEC. **(i)** Relative *Ihh* expression in MODE-K cells after stimulation with the TLR2-agonist peptidoglycan (PG) (n=4 vs 4. p=0.0059). For the qRT-PCR assays, *L32* was used as housekeeping gene. In all panels, the values were normalized for the mean expression of the control group. For panels **a-c, e-i,** individual values are displayed as dots, while mean ± SEM is shown as a column and error bar. For panel **b,** statistical analyses were performed with one-way ANOVA, Tukey's multiple comparison test. For panels **a, c, e-i,** unpaired Student's t test was used. *p<0.05, **p<0.01, ***p<0.001, ****p<0.0001. **(j,k)** smFISH for the Hh-downstream targets *Gli1* (magenta), *Ptch1* (green) and *Hhip* (white) on distal small intestine sections from **(j)** GF vs CONV-R and **(k)** *Tlr2*^{ΔIEC} in comparison to WT littermates. *Gli1* and *Ptch1* transcripts are highlighted with color-coded arrowheads. For each group, the experiment was performed on n=3 mice. For each representative image, two magnifications are shown. Scale bar: 100 µm and 10 µm. For panels **a-c**, **e-h**, **j**, n represents the number of biological independent mice, whereas in panel i it represents the number of independent experiments on cell cultures.

**Figure 2****. NRP-1 protein levels in the gut epithelium are regulated by the gut microbiota through TLR2-mediated lysosomal degradation, (a)** Representative immunofluorescence images of NRP-1 expression (in green) in the distal small intestine of GF and CONV-R mice. The experiment was performed twice. Cell nuclei are counterstained with DAPI (in blue). Scale bar: 100 µm. **(b)** Relative NRP-1 protein levels in the distal small intestine of GF, CONV-R, and CONV-D mice (n=4 vs 5 vs 6. GF vs CONV-D, p=0.0018; GF vs CONV-R, p=0.0001). NRP-1 **(c)** protein (n=5 vs 4) and **(d)** mRNA (n=13 vs 9) expression in IEC isolated from GF and CONV-R mice (p=0.0009) or **(e)** WT mice vs *Tlr2*^{*-*/*-*} in CONV-R housing conditions (n=5 vs 4. p<0.0001). For panels **b-e,** n represents the number of biological independent mice. **(f)** TLR2-mediated NRP-1 degradation in MODE-K cells by lysosome or proteasome. For both panels, NRP-1 degradation by TLR2 is induced by MALP-2 (TLR2/6 agonist) stimulation. Blocking of lysosomal degradation *(leftpanel)* is achieved by stimulation with bafilomycin A1 in 0.125% (v/v) DMSO (Vehicle). Lysosome inhibition prevents TLR2/6-induced NRP-1 degradation (MALP-2 + bafilomycin A1) (control vs MALP-2, p<0.0001; MALP-2 vs MALP-2+bafilomycin A1, p<0.0001; vehicle vs MALP-2+bafilomycin A1, p=0.0122). Blocking of proteasome (*right panel*) is performed by stimulation with epoxomicin in 0.1% (v/v) DMSO (Vehicle). Proteasome inhibition does not prevent NRP-1 degradation by MALP-2 (MALP-2 + epoxomicin) (control vs MALP-2, p=0.0328; vehicle vs MALP-2+epoxomicin, p=0.0340). **(g)** Inhibition of NRP-1 degradation by lysosome shown by flow cytometry, using the same experimental conditions of panel **f** (medium vs MALP-2, p=0.0459; MALP-2 vs MALP-2+bafilomycin A1, p=0.0011). Representative histograms are shown (*right panel*). For WB analyses, the number of independent experiments (n) on cell cultures is 4-8, whereas for flow cytometry n=5. In panel c, GF and CONV-R mice were analyzed on different gels, that were processed in parallel. In the qPCR assay, *L32* was used as the housekeeping gene, whereas in WB protein expression is relative to α-actinin or β-actin. For panels **b-f** the values are normalized for the mean expression of the controls. For panels **b-g,** individual values are displayed as dots, while mean ± SEM is shown as a column and error bar. For panel **b, f-g,** statistical analyses were performed with one-way ANOVA, Tukey's multiple comparison test, whereas for panel **c-e,** Unpaired Student's t test was used. *p<0.05, **p<0.01, ***p<0.001, ****p<0.0001.

**Figure 3****. Impairment of gut barrier function by the gut microbiota, gut epithelial neuropilin-1-deficiency, and inhibition of hedgehog signaling.** FITC-dextran permeability assay on **(a)** GF vs CONV-R mice (n=5 vs 11. p=0.0006); **(h)** WT *(Cre-*negative) littermates vs *NRP-1*^{ΔIEC} in CONV-R status (n=7 vs 5. p=0.0074) and (i) after antibiotics treatment (Abx) (n=8 vs 11); and **(m)** vehicle controls vs GDC-0449-treated mice (n=5 vs 6. p=0.0012). Relative gene expression of claudin-4 *(Cld4),* junctional adhesion molecule-A (*F11r*), occludin *(Ocln),* and zonula occludens-1 *(Tjp1)* in IEC from **(b)** GF vs CONV-R mice (n=6 vs 6. *Cldn4,* p=0.0004; *F11r,* p=0.0015; *Ocln,* p<0.0001; *Tlp1,* p<0.0001), **(j)** WT littermates vs *NRP-1*^{ΔIEC} mice (n=7 vs 7. *Cldn4,* p<0.0001; *F11r,* p=0.0099; *Ocln,* p=0.0019; *Tlp1,* p=0.0003) and **(n)** controls vs GDC-0449-treated mice (n=7vs 7. *Cldn4,* p=0.0010; *F11r,* p=0.0011; *Ocln,* p<0.0001; *Tlp1,* p=0.0010). Relative occludin and zonula occludens-1 (ZO-1) protein expression in IEC of **(c,d)** GF vs CONV-R mice (Occludin, n=13 vs 19. p=0.0015; ZO-1, n= 4 vs 9. p=0.0053), **(k,l)** WT littermates vs *NRP-1*^{ΔIEC} mice (n=7 vs 7. ZO-1, p=0.0337) and **(o,p)** controls vs GDC-0449-treated mice (Occludin, n=7 vs 11. ZO-1, n=7 vs 8). Relative gene expression of *Ihh* and *Gli1* in the distal small intestine of WT littermates vs *NRP-1*^{ΔIEC} mice in **(e)** CONV-R conditions *(Ihh,* n=7 vs 7. p=0.0033; *Gli1,* n=6 vs 7. p=0.0185) and **(f)** after antibiotic treatment (Abx) *(Ihh,* n=7 vs 6. *Gli1,* n=6 vs 6). **(g)** Gut microbiota mean relative abundance on the phylum level as determined by bacterial 16S rRNA gene sequencing of small intestinal tissue samples from *NRP-1*^{ΔIEC} mice vs Cre-negative WT littermates. For all panels, n represents the number of biological independent mice. For the qPCR assays, *L32* was used as the housekeeping gene, while in WB protein expression is relative to α-actinin. In all panels, the values were normalized for the mean of the control group. For panels **q-f, h-p,** individual values are displayed as dots, while mean ± SEM is shown as a column and error bar. For panel **g,** individual values are not shown. For all panels, unpaired Student's t test was used. *p<0.05, **p<0.01, ***p<0.001, ****p<0.0001.

**Figure 4****. Deficiency of epithelial NRP-1 reduces density of blood capillaries without affecting lacteal length in the distal small intestine. (a)** Representative immunofluorescence images of PECAM1 (CD31) expression (in green) in the distal small intestine of mice. The analysis was repeated on n=5 vs 6 mice. **(b)** Quantification of CD31-positive area per villus structure (ROI: region of interest), indicating vessel density (n=5 vs 6. p=0.0013). **(c)** Relative gene expression of *Pecam1* in the distal small intestine (n=6 vs 6. p=0.0103). **(d)** Representative immunofluorescence images of CD31 (green) and LYVE1 (red) in the distal small intestine of mice. The analysis was repeated on n=5 vs 5 mice. Measurements of **(e)** villus length, **(f)** lacteal length and **(g)** the lacteal-to-villus ratio (n=5 vs 5). Semaphorin 3A (SEMA3A) **(h)** protein (n=9 vs 14. p=0.0189) and **(i)** mRNA levels in isolated IEC (n=3 vs 6). For all panels, *NRP-1*^{ΔIEC} mice are compared to floxed WT littermates (Cre-negative). n represents the number of biological independent mice. For immunofluorescence images, cells nuclei are counterstained with To-Pro-3 iodide (in blue). Scale bar 200 µm. For each mouse, the mean measurements of 5-10 villi were taken into account and displayed as a single dot. For the qPCR assays, *L32* was used as the housekeeping gene, while in WB protein expression is relative to α-actinin. For panels **b-c, e-i,** Individual values are displayed as dots, while mean ± SEM is shown as a column and error bar. Independent samples Student's t test, *p<0.05, **p<0.01.

**Figure 5****. Impact of commensals on hedgehog signaling, neuropilin-1 and intestinal epithelial permeability.** Conserved molecular patterns such as bacterial ligands from the resident gut microbiota stimulate TLRs on the intestinal epithelial cells. In turn, TLR2/6 down-regulates NRP-1 protein levels in the epithelial compartment through lysosomal degradation. When NRP-1 is not degraded, it upregulates IHH, which signals from the epithelium to the mesenchymal compartment. **(a)** In the absence of IHH downstream signaling (shown as exemplification in colonized mice), the transmembrane receptor Patched-1 (PTCH1) suppresses the transmembrane protein Smoothened (SMO), resulting in a repressor form of the transcription factor GLI (GLI-R). **(b)** Conversely, after IHH binding to PTCH1 (exemplified in GF mice), repression on SMO is released, yielding the GLI activator (GLI-A) transcription factor, and subsequent transcription of GLI targets involved in hedgehog signaling (i.e., GLI targets 'on'). In germ-free conditions, this pathway results in upregulation of occludin and ZO-1 protein levels, whereas in **(a)** CONV-R housing conditions the epithelial gut barrier is impaired. In the scheme, upregulated (f) proteins are shown in a green font, whereas downregulated (↓) proteins in a red font.

**Figure 6****. Indian Hedgehog expression in monocolonized and TLR-knockout mouse models; generation and characterization of conditional intestinal epithelial *Tlr2* knockout mouse model; Bone morphogenetic protein 4 regulation by gut microbiota through TLR2. (a)** Relative mRNA expression of *Ihh* in the distal small intestine of GF mice compared to ex-GF mice monocolonized with *Bacteroides thetaiotaomicron* (i.e., *B. theta.)* (n=9 vs 9. p<0.0001). **(b,c)** Relative gene expression of *Ihh* in the distal small intestine of WT mice vs **(b)** *Tlr4*^{*-*/*-*} (CONV-R, n=5 vs 5. GF, n=7 vs 6) and **(c)** *Tlr5*^{*-*/*-*} (CONV-R, n=6 vs 6. GF, n=5 vs 6) global knockout mice in CONV-R and GF housing conditions. **(d)** Generation of the conditional intestinal epithelial *Tlr2*-deficient mouse line *(Tlr2*^{ΔIEC})*.* Mice used in the breeding pairs are highlighted by colored squares. P: parental generation; F1, F2, F3: first, second, third filial generation. **(e)** Relative mRNA expression of *Tlr2* in the distal small intestine (n=7 vs 7. p<0.0001) or IEC (n=7 vs 6. p<0.0001) from *Tlr2*^{ΔIEC} vs WT littermates. **(f)** Relative mRNA expression of *Ptch1* and *Hhip* in the distal small intestine of WT littermates vs *Tlr2*^{ΔIEC} mice (n=7 vs 6. *Ptch1,* p=0.0047; *Hhip,* p<0.0001). (**g-i**) Relative mRNA expression of the Hh-target bone morphogenetic protein 4 *(Bmp4)* in the distal small intestine of **(g)** GF vs CONV-R mice (n= 8 vs 5. p=0.0013), (**h**) WT mice vs *Tlr2*^{*-*/*-*}global knockout mice in CONV-R (n=7 vs 7. p=0.0123) and GF (n=7 vs 7) housing conditions and **(i)** WT littermates vs *Tlr2*^{ΔIEC} mice (n=7 vs 6. p=0.0238). For panels **a-c, e-i**, n represents the number of biological independent mice. For the qPCR assays, *L32* was used as the housekeeping gene and the values were normalized for the mean expression of the control group. For panels **a-c, e-i**, Individual values are shown as dots, while mean ± SEM is shown as a column and error bar (except for panel b, due to bimodal distribution), and unpaired Student's t test was used. **p<0.01, ****p<0.0001.

**Figure 7****. NRP-1 and NRP-2 expression in the gut of GF, CONV-D, and CONV-R mice, and MODE-K cell culture model. (a)** Representative immunofluorescence images of NRP-1 expression (in red) in the distal small intestine of GF and CONV-D mice. Cell nuclei are counterstained with DAPI (in blue). Scale bar: 50 µm. The measurements were repeated one time. **(b)** NRP-2 expression in the distal small intestine of GF, CONV-R and CONV-D mice (n=8 vs 10 vs 5). **(c)** NRP-1 expression along the small intestine of GF mice, compared to CONV-R controls (n=1 vs 1). From the proximal to the distal tract, the small intestine (SI) is divided into 8 equally sized segments. For WB analysis, segments 1, 3, 5, 7 were analyzed. **(d)** Protein expression of NRP-1 after stimulation of MODE-K cells with the TLR2/1 agonist Pam₃CSK₄ (n=5 vs 5). For panels **b-c**, n represents the number of biological independent mice, whereas for panel **d**, n is the number of independent experiments performed on cell cultures. For the Western Blot assays, protein expression is relative to α-actinin or β-actin, and the values were normalized for the mean expression of the control group. For panels **b, d,** individual values are shown as dots, whereas mean ± SEM is shown as a column and error bar. For panel **b,** statistical analyses were performed with one-way ANOVA, Tukey's multiple comparison test. For panel **d,** Unpaired Student's t test was used. ns: p>0.05. (**e**) Gating strategy for flow cytometry analysis on MODE-K cells. In the FSC-A vs SSC-A panel, single cells are selected. Gating of EPCAM⁺ NRP-1⁺ DAPI⁻ singlet specific stain (i.e., living IEC expressing NRP-1) are based on the isotype control (upper panel).

**Figure 8****. Characterization of the *Nrp1*^{ΔIEC} mouse model, efficacy of NRP-1-deficiency, NPR2 expression and microbiome analysis. (a)** Generation of the *Nrp1*^{ΔIEC} mouse line. Mice used in the breeding pairs are highlighted by colored squares. P: parental generation; F1, F2, F3: first, second, third filial generation. **(b)** Genotyping of *Villin-Cre* (upper panel) and *Nrp1 fl*/*fl* (lower panel). **(c)** NRP-1 (n=5 vs 6. p=0.0019) and NRP-2 (n=12 vs 11) protein expression in IEC from WT littermates vs *Nrp1*^{ΔIEC} mice. **(d)** Mean relative abundance on the phylum level as determined by bacterial 16S rRNA gene sequencing of intestinal content samples from *Nrp1*^{ΔIEC} mice and floxed WT littermates. **(e)** Principal coordinate analysis (PCoA) based on the Bray-Curtis index and **(f)** α-Diversity (Shannon index) of content samples and of intestine samples of 16S rRNA gene microbiome sequencing from small intestine of *Nrp1*^{ΔIEC} mice vs floxed WT littermates. **(g)** Descriptive statistics (p values) of the bacterial 16S rRNA gene sequencing analysis on *Nrp1*^{ΔIEC} mice vs floxed WT littermates. Differences in Proteobacteria in intestine samples are highlighted by a black box. **(h)** Relative gene expression of *Gli1, Ptch1* and *Hhip* in the distal small intestine of vehicle controls compared to GDC-0449-treated mice (*Gli1,* n= 7 vs 8. p<0.0001; *Ptch1,* n=7 vs 7. p=0.0001; *Hhip,* n= 7 vs 7. p=0.0008). **(i,j)** NRP-1 expression in **(i)** the distal small intestine (n=7 vs 7) and **(j)** isolated IEC (n=7 vs 7) from vehicle controls compared to GDC-0449-treated mice. For panels **c, h-j,** n represents the number of biological independent mice. For the qPCR assays, *L32* was used as the housekeeping gene, whereas in Western Blot, protein expression is relative to α-actinin or β-actin. In panels **c** and **h-j** the values were normalized for the mean expression of the control group. For panels **c, h-j,** individual values are shown as dots, while mean ± SEM is shown as a column and error bar. For panel **d-g,** individual values are not shown. For panel **c, h-j,** unpaired Student's t test was used. **p<0.01, ***p<0.001, ****p<0.0001.

**Figure 9****. VEGF-A expression in the small intestine of the *Nrp1*^{ΔIEC} mouse line and characterization of blood capillaries and lacteals in the distal small intestine of GDC-0449-treated mice, (a)** VEGF-A ELISA in the distal small intestine (n=7 vs 7) and IECs (n=5 vs 8) isolated from WT littermates vs *Nrp1*^{ΔIEC} mice. **(b)** Representative immunofluorescence images of VEGF-A expression (in green) in the distal small intestine of *Nrp1*^{ΔIEC} mice vs WT littermates. The stainings were repeated on n=4 mice. For panels **c-i,** GDC-0449-treated mice are compared to vehicle controls. **(c)** Representative immunofluorescence images of PECAM-1 (CD31) expression (in green) in the distal small intestine of mice (n=12 vs 11). **(d)** Quantification of CD31-positive area per villus structure (ROI: region of interest), representing vessel density (n=12 vs 11). **(e)** Relative mRNA expression of *Pecam1* in the distal small intestine (n=12 vs 11). *L32* was used as the housekeeping gene. **(f)** Representative immunofluorescence images of CD31 (green) and LYVE-1 (red) in the distal small intestine of mice (n=10 vs 10). Measurements of **(g)** villus length (n=10 vs. 10), **(h)** lacteal length (n=5 vs 5) and **(i)** the lacteal-to-villus ratio (n=5 vs. 5). For panels **a, d-i,** n represents the number of biological independent mice and individual values are shown as dots. Mean ± SEM is shown as a column and error bar. For immunofluorescence images, cell nuclei are counterstained with To-Pro-3 iodide (in blue). Scale bar: 100 µm in panel **b** and 200 µm in panels **c, f.** For each mouse, the mean measurements of 5-10 villi were taken into account and displayed as a single dot. Unpaired Student's t test, ns: p>0.05.

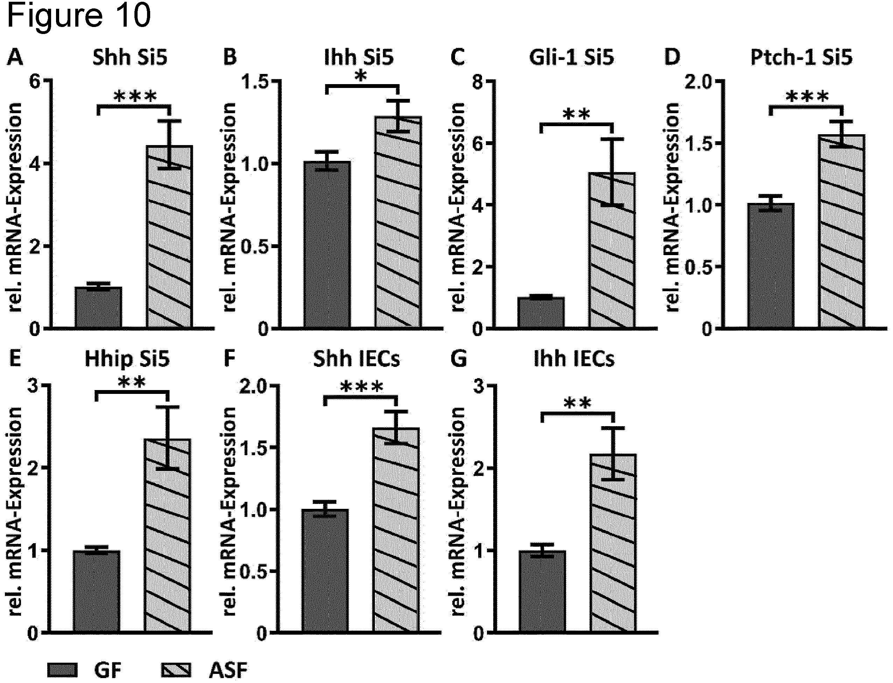

**Figure 10****.** ASF colonization affects mRNA expression of Hh pathway genes. (A-E) mRNA expression of Hh pathway genes in the mid-small intestine (section Si5) of germ-free (GF, n=7) and ASF-colonized (ASF, n=7) mice: (A) Sonic Hedgehog (Shh), (B) Indian Hedgehog (Ihh), (C) glioma associated oncogene transcription factor 1 (Gli-1), (D) Patched-1 (Ptch-1), and (E) Hedgehog Interacting Protein (Hhip). In each case, expression is indicated relative to mRNA expression of the RPL32 gene. (F and G) mRNA expression of Hh ligands (F) Shh and (G) Ihh in isolated intestinal epithelial cells (IECs) from the mid and distal small intestine of germ-free (GF, n=7) and ASF-colonized (ASF, n=7) mice relative to mRNA expression of the RPL32 gene. Both male and female mice aged 10 to 12 weeks were used. Bars indicate the group mean ±S.E.M. Group comparison was performed with a two-tailed unpaired t test: ns, not significant; *=p<0.05; **=p<0.01 ; ***p=<0.001.

## Claims

1. A composition, comprising neuropilin-1 (NRP-1) and/or a compound that maintains or upregulates intestinal epithelial NRP-1 protein levels and promotes Hedgehog (Hh) signaling in the intestinal epithelium for use in the prevention or treatment of a disease that is associated with a weakened intestinal barrier.

2. The composition of claim 1, wherein the NRP-1 in said composition is recombinant NRP-1.

3. The composition of claim 2, wherein the recombinant NRP-1 comprises amino acids 890-902 within the cytoplasmic domain and the cytoplasmic guanosine triphosphatase-activating protein domain of plexins.

4. The composition of anyone of claims 1 to 3, wherein the compound that maintains or upregulates intestinal epithelial NRP-1 protein levels and promotes cell-intrinsic Hh signaling has the property of inhibiting NRP-1 degradation, NRP-1 downregulation or NRP-1 inactivation.

5. The composition of claim 4, wherein the compound that inhibits NRP-1 degradation, NRP-1 downregulation or NRP-1 inactivation is selected from the group consisting of TLR2 inhibitors, lysosomal inhibitors, chloroquine or bafilomycin A1.

6. The composition of claim 4, wherein the compound that inhibits NRP-1 degradation, NRP-1 downregulation or NRP-1 inactivation is selected from the group consisting of antisense-oligonucleotide, siRNA, shRNA, non-coding RNA (ncRNA), miRNA, long non-coding RNA (IncRNA) or aptamer.

7. The composition of claim 4, wherein the compound that inhibits NRP-1 degradation, NRP-1 downregulation or NRP-1 inactivation is an antibody or antibody fragment.

8. The composition of anyone of claims 1 to 7, wherein the composition is formulated as a probiotics, prebiotics or synbiotics.

9. The composition of anyone of claims 1 to 8, wherein the compound that maintains or upregulates intestinal epithelial NRP-1 protein levels and promotes Hedgehog (Hh) signaling in the intestinal epithelium comprises bacteria or microorganisms that activate Hh Signaling, or ameliorate microbiota-induced suppression of Hh signaling and/or reduction of intestinal epithelial NRP-1 protein levels.

10. The composition of anyone of claims 1 to 7, wherein the composition is formulated as a pharmaceutical composition, comprising NRP-1 and/or the compound that maintains or upregulates epithelial NRP-1 protein levels and promotes Hedgehog (Hh) signaling, and at least one pharmaceutically acceptable carrier, diluent, adjuvant and/or excipient.

11. The composition of anyone of claims 1 to 7, wherein the disease that is associated with a weakened intestinal barrier is an inflammatory bowel disease selected from the group consisting of ulcerative colitis, Crohn's disease, celiac disease, diverticulitis, pouchitis, proctitis, mucositis after chemotherapy and radiotherapy, radiation induced enteritis, chronic nonspecific colitis.

12. The composition of anyone of claims 1 to 7, wherein the disease that is associated with a weakened intestinal barrier is a tumor disease selected from the group consisting of colorectal carcinoma.

13. An in vitro-method for the detection of a weakened intestinal barrier in a mammal, comprising the steps:
a. providing a sample of the intestinal epithelium from a donor mammal,
b. measuring the protein or mRNA levels of epithelial neuropilin-1 (NRP-1) in epithelium cells isolated from said sample,
c. correlating the observed NRP-1 levels with the levels observed in control samples from healthy donors, wherein lower NRP-1 levels in the sample from the donor mammal compared to the control samples indicate the presence or predisposition of a weakened intestinal barrier in said mammal.

14. The in-vitro method of claim 13, wherein the sample of the intestinal epithelium is a sample from the small intestinal epithelium.
